(19) Europäisches Patentamt
European Patent Office
Office européen des brevets

(11) EP 3 821 908 A1

(12) **EUROPEAN PATENT APPLICATION**
published in accordance with Art. 153(4) EPC

(43) Date of publication:
**19.05.2021 Bulletin 2021/20**

(21) Application number: **19834023.4**

(22) Date of filing: **10.07.2019**

(51) Int Cl.:
*A61K 45/00* (2006.01) *A61K 39/395* (2006.01)
*A61P 25/02* (2006.01) *A61P 27/02* (2006.01)

(86) International application number:
**PCT/JP2019/027370**

(87) International publication number:
**WO 2020/013238 (16.01.2020 Gazette 2020/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **10.07.2018 US 201862696052 P**

(71) Applicant: **Mitsubishi Tanabe Pharma Corporation**
**Osaka-shi,**
**Osaka 541-8505 (JP)**

(72) Inventors:
- **ISHIDA, Hayato**
**Osaka-shi, Osaka 541-8505 (JP)**
- **ISHIDA, Hirokazu**
**Osaka-shi, Osaka 541-8505 (JP)**
- **PALUMBO, M. Joseph**
**Jersey City, New Jersey 07310 (US)**
- **SASAKI, Atsushi**
**Osaka-shi, Osaka 541-8505 (JP)**

(74) Representative: **Hoffmann Eitle**
**Patent- und Rechtsanwälte PartmbB**
**Arabellastraße 30**
**81925 München (DE)**

# (54) PREVENTION OR TREATMENT METHOD FOR PERIPHERAL NEUROPATHY OR PAIN ACCOMPANYING DISEASE IN WHICH PERIPHERAL NEUROPATHY OR ASTROCYTE DISORDER IS RECOGNIZED

(57) The present invention provides novel methods of preventing or treating peripheral neuropathies, and of preventing or treating pain symptoms associated with diseases having peripheral neuropathies or astrocytic damages, and provides an agent for preventing or treating peripheral neuropathies or pain symptoms associated with diseases having peripheral neuropathies or astrocytic damages, which agent contains an RGM inhibiting substance.

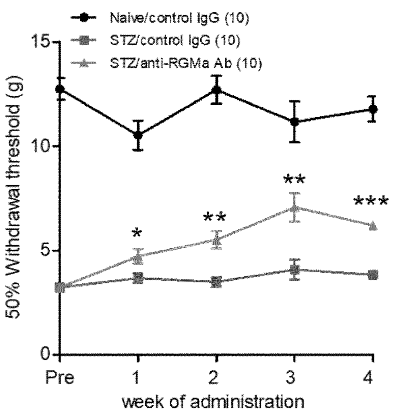


Each data indicates the mean ± S.E.M. (n=10)
Mann-Whitney U test *p<0.05, **p<0.001 vs STZ/control IgG.

Fig. 1



Printed by Jouve, 75001 PARIS (FR)

SEQUENCE LISTING

<110>  Mitsubishi Tanabe Pharma Corporation

<120>  PREVENTION OR TREATMENT METHOD FOR PERIPHERAL NEUROPATHY OR PAIN ACCOMPANYING DISEASE IN WHICH PERIPHERAL NEUROPATHY OR ASTROCYTE DISORDER IS RECOGNIZED

<130>  A19007-18289

<150>  US 62/696,052
<151>  2018-07-10

<160>  45

<170>  PatentIn version 3.5

<210>  1
<211>  450
<212>  PRT
<213>  Homo sapiens

<400>  1

```
Met Gln Pro Pro Arg Glu Arg Leu Val Val Thr Gly Arg Ala Gly Trp
1               5                   10                  15
Met Gly Met Gly Arg Gly Ala Gly Arg Ser Ala Leu Gly Phe Trp Pro
            20                  25                  30
Thr Leu Ala Phe Leu Leu Cys Ser Phe Pro Ala Ala Thr Ser Pro Cys
        35                  40                  45
Lys Ile Leu Lys Cys Asn Ser Glu Phe Trp Ser Ala Thr Ser Gly Ser
    50                  55                  60
His Ala Pro Ala Ser Asp Asp Thr Pro Glu Phe Cys Ala Ala Leu Arg
65                  70                  75                  80
Ser Tyr Ala Leu Cys Thr Arg Arg Thr Ala Arg Thr Cys Arg Gly Asp
                85                  90                  95
Leu Ala Tyr His Ser Ala Val His Gly Ile Glu Asp Leu Met Ser Gln
            100                 105                 110
His Asn Cys Ser Lys Asp Gly Pro Thr Ser Gln Pro Arg Leu Arg Thr
        115                 120                 125
Leu Pro Pro Ala Gly Asp Ser Gln Glu Arg Ser Asp Ser Pro Glu Ile
    130                 135                 140
Cys His Tyr Glu Lys Ser Phe His Lys His Ser Ala Thr Pro Asn Tyr
145                 150                 155                 160
Thr His Cys Gly Leu Phe Gly Asp Pro His Leu Arg Thr Phe Thr Asp
                165                 170                 175
Arg Phe Gln Thr Cys Lys Val Gln Gly Ala Trp Pro Leu Ile Asp Asn
            180                 185                 190
Asn Tyr Leu Asn Val Gln Val Thr Asn Thr Pro Val Leu Pro Gly Ser
        195                 200                 205
Ala Ala Thr Ala Thr Ser Lys Leu Thr Ile Ile Phe Lys Asn Phe Gln
    210                 215                 220
Glu Cys Val Asp Gln Lys Val Tyr Gln Ala Glu Met Asp Glu Leu Pro
225                 230                 235                 240
Ala Ala Phe Val Asp Gly Ser Lys Asn Gly Gly Asp Lys His Gly Ala
                245                 250                 255
Asn Ser Leu Lys Ile Thr Glu Lys Val Ser Gly Gln His Val Glu Ile
            260                 265                 270
Gln Ala Lys Tyr Ile Gly Thr Thr Ile Val Val Arg Gln Val Gly Arg
        275                 280                 285
Tyr Leu Thr Phe Ala Val Arg Met Pro Glu Glu Val Val Asn Ala Val
    290                 295                 300
Glu Asp Trp Asp Ser Gln Gly Leu Tyr Leu Cys Leu Arg Gly Cys Pro
```

```
305                 310                 315                 320
Leu Asn Gln Gln Ile Asp Phe Gln Ala Phe His Thr Asn Ala Glu Gly
                325                 330                 335
Thr Gly Ala Arg Arg Leu Ala Ala Ala Ser Pro Ala Pro Thr Ala Pro
            340                 345                 350
Glu Thr Phe Pro Tyr Glu Thr Ala Val Ala Lys Cys Lys Glu Lys Leu
        355                 360                 365
Pro Val Glu Asp Leu Tyr Tyr Gln Ala Cys Val Phe Asp Leu Leu Thr
    370                 375                 380
Thr Gly Asp Val Asn Phe Thr Leu Ala Ala Tyr Tyr Ala Leu Glu Asp
385                 390                 395                 400
Val Lys Met Leu His Ser Asn Lys Asp Lys Leu His Leu Tyr Glu Arg
                405                 410                 415
Thr Arg Asp Leu Pro Gly Arg Ala Ala Ala Gly Leu Pro Leu Ala Pro
            420                 425                 430
Arg Pro Leu Leu Gly Ala Leu Val Pro Leu Leu Ala Leu Leu Pro Val
        435                 440                 445
Phe Cys
    450


<210>  2
<211>  454
<212>  PRT
<213>  Mus musculus

<400>  2

Met Gln Pro Pro Arg Glu Arg Leu Val Val Thr Gly Arg Ala Gly Trp
1               5                   10                  15
Met Gly Met Gly Arg Gly Ala Gly Arg Ser Ala Leu Gly Leu Trp Pro
            20                  25                  30
Thr Leu Ala Phe Leu Leu Cys Ser Phe Pro Ala Ala Ile Ser Pro Cys
        35                  40                  45
Lys Ile Leu Lys Cys Asn Ser Glu Phe Trp Ser Ala Thr Ser Ser Gly
    50                  55                  60
Ser His Ala Pro Ala Ser Asp Asp Val Pro Glu Phe Cys Ala Ala Leu
65                  70                  75                  80
Arg Thr Tyr Ala Leu Cys Thr Arg Arg Thr Ala Arg Thr Cys Arg Gly
                85                  90                  95
Asp Leu Ala Tyr His Ser Ala Val His Gly Ile Glu Asp Leu Met Ser
            100                 105                 110
Gln His Asn Cys Ser Lys Asp Gly Pro Thr Ser Gln Pro Arg Val Arg
        115                 120                 125
Thr Leu Pro Pro Ala Gly Asp Ser Gln Glu Arg Ser Asp Ser Pro Glu
    130                 135                 140
Ile Cys His Tyr Glu Lys Ser Phe His Lys His Ser Ala Ala Pro Asn
145                 150                 155                 160
Tyr Thr His Cys Gly Leu Phe Gly Asp Pro His Leu Arg Thr Phe Thr
                165                 170                 175
Asp His Phe Gln Thr Cys Lys Val Gln Gly Ala Trp Pro Leu Ile Asp
            180                 185                 190
Asn Asn Tyr Leu Asn Val Gln Val Thr Asn Thr Pro Val Leu Pro Gly
        195                 200                 205
Ser Ala Ala Thr Ala Thr Ser Lys Leu Thr Ile Ile Phe Lys Asn Phe
    210                 215                 220
Gln Glu Cys Val Asp Gln Lys Val Tyr Gln Ala Glu Met Asp Glu Leu
225                 230                 235                 240
Pro Ser Ala Phe Ala Asp Gly Ser Lys Asn Gly Gly Asp Lys His Gly
                245                 250                 255
Ala Asn Ser Leu Lys Ile Thr Glu Lys Val Ser Gly Gln His Val Glu
            260                 265                 270
Ile Gln Ala Lys Tyr Ile Gly Thr Thr Ile Val Val Arg Gln Val Gly
```

```
        275                 280                 285
Arg Tyr Leu Thr Phe Ala Val Arg Met Pro Glu Glu Val Val Asn Ala
    290                 295                 300
Val Glu Asp Arg Asp Ser Gln Gly Leu Tyr Leu Cys Leu Arg Gly Cys
305                 310                 315                 320
Pro Leu Asn Gln Gln Ile Asp Phe Gln Ala Phe Arg Ala Asn Ala Glu
                325                 330                 335
Ser Pro Arg Arg Pro Ala Ala Ala Ser Pro Ser Pro Val Val Pro Glu
            340                 345                 350
Thr Phe Pro Tyr Glu Thr Ala Val Ala Lys Cys Lys Glu Lys Leu Pro
        355                 360                 365
Val Glu Asp Leu Tyr Tyr Gln Ala Cys Val Phe Asp Leu Leu Thr Thr
    370                 375                 380
Gly Asp Val Asn Phe Thr Leu Ala Ala Tyr Tyr Ala Leu Glu Asp Gly
385                 390                 395                 400
Lys Met Leu His Ser Asn Lys Asp Lys Leu His Leu Phe Glu Arg Thr
                405                 410                 415
Arg Glu Leu Pro Gly Ala Val Ala Ala Ala Ala Ala Ala Ala Thr Thr
            420                 425                 430
Phe Pro Leu Ala Pro Gln Ile Leu Leu Gly Thr Ile Pro Leu Leu Val
        435                 440                 445
Leu Leu Pro Val Leu Trp
    450


<210>  3
<211>  449
<212>  PRT
<213>  Rattus norvegicus

<400>  3

Met Gln Pro Pro Arg Glu Arg Leu Val Val Thr Gly Arg Ala Gly Trp
1               5                   10                  15
Met Gly Met Gly Arg Gly Ala Gly Arg Ser Ala Leu Gly Leu Trp Pro
            20                  25                  30
Thr Leu Ala Phe Leu Leu Cys Ser Phe Pro Ala Ala Ile Ser Pro Cys
        35                  40                  45
Lys Ile Leu Lys Cys Asn Ser Glu Phe Trp Ser Ala Thr Ser Ser Gly
    50                  55                  60
Ser His Ala Pro Ala Ser Asp Asp Val Pro Glu Phe Cys Ala Ala Leu
65                  70                  75                  80
Arg Thr Tyr Ala Leu Cys Thr Arg Arg Thr Ala Arg Thr Cys Arg Gly
                85                  90                  95
Asp Leu Ala Tyr His Ser Ala Val His Gly Ile Glu Asp Leu Met Ser
            100                 105                 110
Gln His Asn Cys Ser Lys Asp Gly Pro Thr Ser Gln Pro Arg Val Arg
        115                 120                 125
Thr Leu Pro Pro Ala Gly Asp Ser Gln Glu Arg Ser Asp Ser Pro Glu
    130                 135                 140
Ile Cys His Tyr Glu Lys Ser Phe His Lys His Ser Ala Ala Pro Asn
145                 150                 155                 160
Tyr Thr His Cys Gly Leu Phe Gly Asp Pro His Leu Arg Thr Phe Thr
                165                 170                 175
Asp His Phe Gln Thr Cys Lys Val Gln Gly Ala Trp Pro Leu Ile Asp
            180                 185                 190
Asn Asn Tyr Leu Asn Val Gln Val Thr Asn Thr Pro Val Leu Pro Gly
        195                 200                 205
Ser Ala Ala Thr Ala Thr Ser Lys Leu Thr Ile Ile Phe Lys Asn Phe
    210                 215                 220
Gln Glu Cys Val Asp Gln Lys Val Tyr Gln Ala Glu Met Asp Glu Leu
225                 230                 235                 240
Pro Ser Ala Phe Ala Asp Gly Ser Lys Asn Gly Gly Asp Lys His Gly
```

```
                245                 250                 255
Ala Asn Ser Leu Lys Ile Thr Glu Lys Val Ser Gly Gln His Val Glu
            260                 265                 270
Ile Gln Ala Lys Tyr Ile Gly Thr Thr Ile Val Val Arg Gln Val Gly
        275                 280                 285
Arg Tyr Leu Thr Phe Ala Val Arg Met Pro Glu Glu Val Val Asn Ala
    290                 295                 300
Val Glu Asp Arg Asp Ser Gln Gly Leu Tyr Leu Cys Leu Arg Gly Cys
305                 310                 315                 320
Pro Leu Asn Gln Gln Ile Asp Phe Gln Ala Phe Arg Ala Asn Ala Glu
                325                 330                 335
Ser Pro Arg Arg Pro Ala Ala Ala Ser Pro Ser Pro Val Val Pro Glu
            340                 345                 350
Thr Phe Pro Tyr Glu Thr Ala Val Ala Lys Cys Lys Glu Lys Leu Pro
        355                 360                 365
Val Glu Asp Leu Tyr Tyr Gln Ala Cys Val Phe Asp Leu Leu Thr Thr
    370                 375                 380
Gly Asp Val Asn Phe Thr Leu Ala Ala Tyr Tyr Ala Leu Glu Asp Gly
385                 390                 395                 400
Lys Met Leu His Ser Asn Lys Asp Lys Leu His Leu Phe Glu Arg Thr
                405                 410                 415
Arg Glu Leu Pro Gly Ala Val Ala Ala Ala Ala Phe Pro Leu Ala Pro
            420                 425                 430
Glu Met Leu Pro Gly Thr Val Thr Leu Leu Val Leu Leu Pro Leu Phe
        435                 440                 445
Trp
```

<210>  4
<211>  1353
<212>  DNA
<213>  Homo sapiens

<400>  4

```
atgcagccgc caagggagag gctagtggta acaggccgag ctggatggat gggtatgggg      60
agaggggcag gacgttcagc cctgggattc tggccgaccc tcgccttcct tctctgcagc     120
ttccccgcag ccacctcccc gtgcaagatc ctcaagtgca actctgagtt ctggagcgcc     180
acgtcgggca gccacgcccc agcctcagac gacacccccg agttctgtgc agccttgcgc     240
agctacgccc tgtgcacgcg gcggacggcc cgcacctgcc ggggtgacct ggcctaccac     300
tcggccgtcc atggcataga ggacctcatg agccagcaca actgctccaa ggatggcccc     360
acctcgcagc cacgcctgcg cacgctccca ccggccggag acagccagga gcgctcggac     420
agccccgaga tctgccatta cgagaagagc tttcacaagc actcggccac ccccaactac     480
acgcactgtg gcctcttcgg ggacccacac ctcaggactt tcaccgaccg cttccagacc     540
tgcaaggtgc agggcgcctg gccgctcatc gacaataatt acctgaacgt gcaggtcacc     600
aacacgcctg tgctgcccgg ctcagcggcc actgccacca gcaagctcac catcatcttc     660
aagaacttcc aggagtgtgt ggaccagaag gtgtaccagg ctgagatgga cgagctcccg     720
gccgccttcg tggatggctc taagaacggt ggggacaagc acggggccaa cagcctgaag     780
atcactgaga aggtgtcagg ccagcacgtg gagatccagg ccaagtacat cggcaccacc     840
```

atcgtggtgc gccaggtggg ccgctacctg acctttgccg tccgcatgcc agaggaagtg 900

gtcaatgctg tggaggactg ggacagccag ggtctctacc tctgcctgcg gggctgcccc 960

ctcaaccagc agatcgactt ccaggccttc cacaccaatg ctgagggcac cggtgcccgc 1020

aggctggcag ccgccagccc tgcacccaca gcccccgaga ccttcccata cgagacagcc 1080

gtggccaagt gcaaggagaa gctgccggtg gaggacctgt actaccaggc ctgcgtcttc 1140

gacctcctca ccacgggcga cgtgaacttc acactggccg cctactacgc gttggaggat 1200

gtcaagatgc tccactccaa caaagacaaa ctgcacctgt atgagaggac tcgggacctg 1260

ccaggcaggg cggctgcggg gctgcccctg gcccccggc ccctcctggg cgccctcgtc 1320

ccgctcctgg ccctgctccc tgtgttctgc tag 1353

<210> 5
<211> 11
<212> PRT
<213> Mus musculus

<400> 5

Arg Ala Ser Gln Asp Ile Ser Ser Tyr Leu Asn
1 5 10

<210> 6
<211> 7
<212> PRT
<213> Mus musculus

<400> 6

Tyr Thr Ser Arg Leu His Ser
1 5

<210> 7
<211> 7
<212> PRT
<213> Mus musculus

<400> 7

Gln Gln Leu Asn Thr Leu Pro
1 5

<210> 8
<211> 5
<212> PRT
<213> Mus musculus

<400> 8

Asp Ala Trp Met Asp
1 5

<210> 9
<211> 19
<212> PRT
<213> Mus musculus

<400> 9

Glu Ile Arg Ser Lys Ala Asn Asn His Ala Thr Tyr Tyr Ala Glu Ser
1 5 10 15

Val Lys Gly

<210> 10
<211> 5
<212> PRT
<213> Mus musculus

<400> 10

Arg Asp Gly Ala Tyr
1 5

<210> 11
<211> 16
<212> PRT
<213> Mus musculus

<400> 11

Arg Ser Ser Gln Ser Leu Val His Ser Asn Gly Asn Thr Tyr Leu His
1 5 10 15

<210> 12
<211> 7
<212> PRT
<213> Mus musculus

<400> 12

Lys Val Ser Asn Arg Phe Ser
1 5

<210> 13
<211> 7
<212> PRT
<213> Mus musculus

<400> 13

Ser Gln Ser Thr His Val Pro
1 5

<210> 14
<211> 6
<212> PRT
<213> Mus musculus

<400>  14

Thr Ser Tyr Tyr Trp Asn
1               5

<210>  15
<211>  16
<212>  PRT
<213>  Mus musculus

<400>  15

Tyr Ile Ser Tyr Asp Gly Thr Asn Asn Tyr Asn Pro Ser Leu Lys Asn
1               5                   10                  15

<210> 16
<211> 23
<212> PRT
<213> Homo sapiens

<400> 16

Pro Cys Lys Ile Leu Lys Cys Asn Ser Glu Phe Trp Ser Ala Thr Ser
1               5                   10                  15

Gly Ser His Ala Pro Ala Ser
            20

<210>  17
<211>  16
<212>  PRT
<213>  Artificial Sequence

<220>
<223>  Description of Artificial Sequence: Synthetic
       peptide

<400>  17

Arg Ser Ser Gln Ser Leu Glu Tyr Ser Asp Gly Tyr Thr Phe Leu Glu
1               5                   10                  15

<210>  18
<211>  7
<212>  PRT
<213>  Artificial Sequence

<220>
<223>  Description of Artificial Sequence: Synthetic
       peptide

<400>  18

Glu Val Ser Asn Arg Phe Ser
1               5

<210> 19
<211> 9
<212> PRT
<213> Artificial Sequence

<220>
<223> Description of Artificial Sequence: Synthetic peptide

<400> 19

Phe Gln Ala Thr His Asp Pro Leu Thr
1 5

<210> 20
<211> 5
<212> PRT
<213> Artificial Sequence

<220>
<223> Description of Artificial Sequence: Synthetic peptide

<400> 20

Asn Tyr Gly Met Asn
1 5

<210> 21
<211> 17
<212> PRT
<213> Artificial Sequence

<220>
<223> Description of Artificial Sequence: Synthetic peptide

<400> 21

Met Ile Tyr Tyr Asp Ser Ser Glu Lys His Tyr Ala Asp Ser Val Lys
1 5 10 15

Gly

<210> 22
<211> 6
<212> PRT
<213> Artificial Sequence

<220>
<223> Description of Artificial Sequence: Synthetic peptide

<400> 22

Gly Thr Thr Pro Asp Tyr
1 5

<210> 23
<211> 11
<212> PRT
<213> Artificial Sequence

<220>
<223> Description of Artificial Sequence: Synthetic peptide

<400> 23

Gln Ala Ser Gln Asp Ile Asp Asn Tyr Leu Ala
1 5 10

<210> 24
<211> 7
<212> PRT
<213> Artificial Sequence

<220>
<223> Description of Artificial Sequence: Synthetic peptide

<400> 24

Gly Ala Thr Asn Leu Ala Asp
1 5

<210> 25
<211> 9
<212> PRT
<213> Artificial Sequence

<220>
<223> Description of Artificial Sequence: Synthetic peptide

<400> 25

Leu Gln Gly Tyr Ile Pro Pro Arg Thr
1 5

<210> 26
<211> 5
<212> PRT
<213> Artificial Sequence

<220>
<223> Description of Artificial Sequence: Synthetic peptide

<400> 26

Ser Tyr Val Met His
1 5

<210> 27
<211> 17

```
<212>  PRT
<213>  Artificial Sequence

<220>
<223>  Description of Artificial Sequence: Synthetic
       peptide

<400>  27

Tyr Ile Ile Pro Tyr Asn Asp Asn Thr Lys Tyr Asn Glu Lys Phe Lys
1               5                   10                  15


Gly


<210>  28
<211>  14
<212>  PRT
<213>  Artificial Sequence

<220>
<223>  Description of Artificial Sequence: Synthetic
       peptide

<400>  28

Ala Arg Arg Asn Glu Tyr Tyr Gly Ser Ser Phe Phe Asp Tyr
1               5                   10


<210> 29
<211> 14
<212> PRT
<213> Artificial Sequence

<220>
<223> Description of Artificial Sequence: Synthetic
      peptide

<400> 29

Thr Gly Thr Ser Ser Ser Val Gly Asp Ser Ile Tyr Val Ser
1               5                   10


<210> 30
<211> 7
<212> PRT
<213> Artificial Sequence

<220>
<223> Description of Artificial Sequence: Synthetic
      peptide

<400> 30

Asp Val Thr Lys Arg Pro Ser
1               5


<210> 31
<211> 9
```

<212> PRT
<213> Artificial Sequence

<220>
<223> Description of Artificial Sequence: Synthetic
peptide

<400> 31

Cys Ser Tyr Ala Gly Thr Asp Thr Leu
1 5

<210> 32
<211> 5
<212> PRT
<213> Artificial Sequence

<220>
<223> Description of Artificial Sequence: Synthetic
peptide

<400> 32

Ser His Gly Ile Ser
1 5

<210> 33
<211> 17
<212> PRT
<213> Artificial Sequence

<220>
<223> Description of Artificial Sequence: Synthetic
peptide

<400> 33

Trp Ile Ser Pro Tyr Ser Gly Asn Thr Asn Tyr Ala Gln Lys Leu Gln
1 5 10 15

Gly

<210> 34
<211> 11
<212> PRT
<213> Artificial Sequence

<220>
<223> Description of Artificial Sequence: Synthetic
peptide

<400> 34

Val Gly Ser Gly Pro Tyr Tyr Tyr Met Asp Val
1 5 10

<210> 35

<211> 9
<212> PRT
<213> Artificial Sequence

<220>
<223> Description of Artificial Sequence: Synthetic peptide

<400> 35

Tyr Ser Tyr Ala Gly Thr Asp Thr Leu
1               5

<210> 36
<211> 14
<212> PRT
<213> Homo sapiens

<400> 36

Glu Glu Val Val Asn Ala Val Glu Asp Trp Asp Ser Gln Gly
1               5                   10

<210> 37
<211> 14
<212> PRT
<213> Homo sapiens

<400> 37

Asn Gln Gln Ile Asp Phe Gln Ala Phe His Thr Asn Ala Glu
1               5                   10

<210> 38
<211> 11
<212> PRT
<213> Homo sapiens

<400> 38

Pro Thr Ala Pro Glu Thr Phe Pro Tyr Glu Thr
1               5                   10

<210> 39
<211> 11
<212> PRT
<213> Homo sapiens

<400> 39

Lys Leu Pro Val Glu Asp Leu Tyr Tyr Gln Ala
1               5                   10

<210> 40
<211> 9
<212> PRT
<213> Artificial Sequence

<220>
<223> Description of Artificial Sequence: Synthetic
peptide

<400> 40
Phe Ser Tyr Ala Gly Thr Asp Thr Leu
1 5

<210> 41
<211> 9
<212> PRT
<213> Artificial Sequence

<220>
<223> Description of Artificial Sequence: Synthetic
peptide

<400> 41
His Ser Tyr Ala Gly Thr Asp Thr Leu
1 5

<210> 42
<211> 9
<212> PRT
<213> Artificial Sequence

<220>
<223> Description of Artificial Sequence: Synthetic
peptide

<400> 42
Leu Ser Tyr Ala Gly Thr Asp Thr Leu
1 5

<210> 43
<211> 9
<212> PRT
<213> Artificial Sequence

<220>
<223> Description of Artificial Sequence: Synthetic
peptide

<400> 43
Val Ser Tyr Ala Gly Thr Asp Thr Leu
1 5

<210> 44
<211> 9
<212> PRT
<213> Artificial Sequence

<220>
<223> Description of Artificial Sequence: Synthetic
peptide

<400> 44
Ile Ser Tyr Ala Gly Thr Asp Thr Leu
1 5

```
<210> 45
<211> 9
<212> PRT
<213> Artificial Sequence

<220>
<223> Description of Artificial Sequence: Synthetic
      peptide

<400> 45
Lys Ser Tyr Ala Gly Thr Asp Thr Leu
1               5
```

**Description**

TECHNICAL FIELD

The present invention relates to an agent for preventing or treating peripheral neuropathies or pains associated with diseases having peripheral neuropathies or astrocytic damages, and a method of preventing or treating them using the agent, the method characterized by inhibiting an RGMa activity.

BACKGROUND ART

Astrocytes, one of glia cells present in central nervous systems, have various functions such as (1) a function to structurally support the network of neurons; (2) a function to regulate various conditions around the astrocytes through transportation of substances; (3) a single synaptic function exerted by three types of cells through close relationships among presynaptic, postsynaptic and glia cells; (4) regulation of extracellular ion concentration; (5) buffering action in energetic aspects; and (6) enhancement of myelination activity of oligodendrocyte. The functions of astrocytes as described above in various brain diseases, especially, the relationship of neurodegenerative diseases such as Alzheimer's disease, Parkinson's disease and cognitive impairment, cerebrovascular accidents, and mental illnesses by astrocytic hypofunctions associated with aging have been attracting attention (Non-patent Documents 1 to 3).

Peripheral neuropathies are pathological conditions in which normal conduction of peripheral nerves is impaired. In peripheral neuropathies, nerves such as motor nerves, sensory nerves, and autonomic nerves are impaired. Peripheral neuropathies are classified clinically into mononeuropathies (disorders in a single nerve), mononeuropathy multiplexes (disorders in two or more nerves present in separate regions), or polyneuropathies (symmetrically and extensively developed neuropathies), or pathologically into axonopathies in which axons are mainly affected or myelinopathies in which myelin sheaths are denatured and detached. Peripheral neuropathies lead to dysfunctions of motor nerves, sensory nerves, and autonomic nerves. This causes symptoms such as pain, dysesthesia, paralysis, numbness, muscle weakness, dyshidrosis, and dysuria, which become worse with time. Peripheral neuropathies are mainly caused by, for example, injuries due to physical factors such as deformation; compressions, disruptions of blood circulation, genetic factors, and metabolic abnormalities. Diabetic neuropathies include polyneuropathies and mononeuropathies. Polyneuropathies include neuropathies of peripheral nerves, sensory nerves, motor nerves and autonomic nerves.

Methyl vitamin B 12 (methylcobalamin), as a therapeutic agent for peripheral neuropathies, is now clinically used (Non-patent Document 4), but is clinically effective only in very rare cases. Furthermore, for motor paralysis associated with neuropathies, no effective therapy has yet been established though it seriously affects daily living.

RGM (repulsive guidance molecule) is a membrane protein that has been initially identified as an axon guidance molecule in the visual system (see Non-patent Document 5). RGM family include three members called RGMa, RGMb and RGMc (Non-patent Document 6). At least RGMa and RGMb are known to work in the same signaling mechanism (see Non-patent Document 7). RGMc plays an important role in iron metabolisms.

Subsequent studies have revealed that RGM functions to control, for example, axon guidance and laminar formation in Xenopus and chick embryos, and cephalic neural tube closure in mouse embryos (see Non-patent Document 8). Patent Document 1 discloses an axon regeneration promoting agent containing an anti-RGM neutralizing antibody as an active ingredient.

In addition to its functions in developmental stages, RGMa is expressed again after central nervous system injuries in an adult human and rat. Further, inhibition of RGMa in rat promotes axon growth after spinal cord injuries and facilitates functional recovery (see Non-Patent Literature 9). From these facts, RGMa is considered as an inhibitor of axon regeneration after central nervous system injuries. Specific examples of antibodies to neutralize RGMa are described in Patent Document 2 (such as 5F9, and 8D1), Patent Document 3 (such as AE12-1, an AE12-1Y), and Patent Document 4 (such as r1 16A3, r70E4, r1 16A3C, and rH1 16A3).

It has also been known that anti-RGMa antibodies are effective for neuromyelitis optica (see Non-patent Document 10).

As described above, roles of RGMa in central nervous system injuries have already been revealed, but the role of RGMa have not yet been identified in peripheral neuropathies or pain symptoms associated with diseases having peripheral neuropathies or astrocytic damages.

[Prior Arts]

[Patent Documents]

Patent Document 1: WO2005/087268
Patent Document 2: WO2009/106356
Patent Document 3: WO2013/112922
Patent Document 4: WO2016/175236

[Non-patent Documents]

Non-patent Document 1: Dallerac Get al., Prog Neurobiol. 144: 48-67(2016)
Non-patent Document 2: Gerkau NJ et al., J Neurosci Res. 2017 Feb 2.
Non-patent Document 3: Alam Q et al., Curr Pharm Des. 22: 541-8(2016)
Non-patent Document 4: Yamazaki et al., Neurosci Lett, 170: 195-197. (1994)
Non-patent Document 5: Stahl, B., Muller, B., von Boxberg, Y., Cox, E.C. & Bonhoeffer, F. Biochemical characterization of a putative axonal guidance molecule of the chick visual system. Neuron 5, 735-743 (1990)
Non-patent Document 6: Mueller et al., Philos. Trans. R. Soc. Lond. B Biol. Sci., 361: 1513 - 29, 2006
Non-patent Document 7: Liu, X., Hashimoto, M., Horii, H., Yamaguchi, A., Naito, K. and Yamashita, T. Repulsive guidance molecule b inhibits neurite growth and is increased after spinal cord injury. Biochem. Biophys. Res. Commun. 382, 795-800 (2009)
Non-patent Document 8: Yamashita, T., Mueller, B.K. & Hata, K. Neogenin and repulsive guidance molecule signaling in the central nervous system. Curr. Opin. Neurobiol.17, 29-34 (2007)
Non-patent Document 9: Hata, K. et al. RGMa inhibition promotes axonal growth and recovery after spinal cord injury. J. Cell Biol. 173, 47-58 (2006)
Non-patent Document 10: Harada K. et al., Inhibition of RGMa alleviates symptoms in a rat model of neuromyelitis optica. Scientific Reports 8:34 1-9 (2018)

SUMMARY OF THE INVENTION

[Problems to be Solved by the Invention]

An object of the present invention is to provide a novel method of preventing or treating peripheral neuropathies or pain symptoms associated with diseases having peripheral neuropathies or astrocytic damages.

[Means to Solve the Problem]

In order to solve the above problems, the present inventors have intensively studied and found that RGMa inhibiting substances can be agents for preventing or treating peripheral neuropathies. The present inventors have also found that RGMa inhibiting substances have effects on pain symptoms through amelioration of not only peripheral neuropathies but also astrocytic damages or hypofunctions, which indicates that RGMa inhibiting substances can also be agents for preventing or treating pain symptoms associated with diseases having peripheral neuropathies or astrocytic damages, and thereby completed the present invention.
Thus, the present invention relates to the followings.

[1] An agent for preventing or treating a peripheral neuropathy, which agent comprises an RGM inhibiting substance.
[2] The agent according to [1], wherein the RGM inhibitor is an RGMa inhibiting substance.
[3] The agent according to [2], wherein the RGMa inhibiting substance is an anti-RGMa neutralizing antibody or a fragment thereof.
[4] The agent according to [3], wherein the anti-RGMa neutralizing antibody is a humanized antibody.
[5] The agent according to [3] or [4], wherein the anti-RGMa neutralizing antibody is an antibody recognizing an amino acid sequence selected from the group consisting of SEQ ID NOS: 16, 36, 37, 38 and 39.
[6] The agent according to any one of [3] to [5], wherein the anti-RGMa neutralizing antibody is an antibody selected from the group consisting of:

(a1) an anti-RGMa neutralizing antibody which comprises
a light chain variable region comprising an LCDR1 comprising the amino acid sequence represented by SEQ ID NO: 5, an LCDR2 comprising the amino acid sequence represented by SEQ ID NO: 6 and an LCDR3 comprising the amino acid sequence represented by SEQ ID NO: 7, and
a heavy chain variable region comprising an HCDR1 comprising the amino acid sequence represented by SEQ ID NO: 8, an HCDR2 comprising the amino acid sequence represented by SEQ ID NO: 9 and an HCDR3 comprising the amino acid sequence represented by SEQ ID NO: 10;

(b 1) an anti-RGMa neutralizing antibody which comprises

a light chain variable region comprising an LCDR1 comprising the amino acid sequence represented by SEQ ID NO: 11, an LCDR2 comprising the amino acid sequence represented by SEQ ID NO: 12 and an LCDR3 comprising the amino acid sequence represented by SEQ ID NO: 13, and

a heavy chain variable region comprising an HCDR1 comprising the amino acid sequence represented by SEQ ID NO: 14, an HCDR2 comprising the amino acid sequence represented by SEQ ID NO: 15 and an HCDR3 comprising an amino acid sequence comprising SFG;

(c1) an anti-RGMa neutralizing antibody which comprises

a light chain variable region comprising an LCDR1 comprising the amino acid sequence represented by SEQ ID NO: 17, an LCDR2 comprising the amino acid sequence represented by SEQ ID NO: 18 and an LCDR3 comprising the amino acid sequence represented by SEQ ID NO: 19, and

a heavy chain variable region comprising an HCDR1 comprising the amino acid sequence represented by SEQ ID NO: 20, an HCDR2 comprising the amino acid sequence represented by SEQ ID NO: 21 and an HCDR3 comprising the amino acid sequence represented by SEQ ID NO: 22;

(d1) an anti-RGMa neutralizing antibody which comprises

a light chain variable region comprising an LCDR1 comprising the amino acid sequence represented by SEQ ID NO: 23, an LCDR2 comprising the amino acid sequence represented by SEQ ID NO: 24 and an LCDR3 comprising the amino acid sequence represented by SEQ ID NO: 25, and

a heavy chain variable region comprising an HCDR1 comprising the amino acid sequence represented by SEQ ID NO: 26, an HCDR2 comprising the amino acid sequence represented by SEQ ID NO: 27 and an HCDR3 comprising the amino acid sequence represented by SEQ ID NO: 28;

(e1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 31, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(f1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 35, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(g1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 40, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(h1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 41, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(i1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 42, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(j1) an anti-RGMa neutralizing antibody which comprises
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 43, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;
(k1) an anti-RGMa neutralizing antibody which comprises
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 44, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34; and
(11) an anti-RGMa neutralizing antibody which comprises
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34.

[7] The agent according to any one of [1] to [6], wherein the peripheral neuropathy is selected from the group consisting of a diabetic neuropathy; entrapment neuropathy such as carpal tunnel syndrome, cubital ulnar neuropathy, peroneal nerve palsy and tarsal tunnel syndrome; familial amyloid polyneuropathy; toxic neuropathy; carcinomatous neuropathy; immune-mediated neuropathy such as Guillain-Barre syndrome (GBS) and chronic inflammatory demyelinating polyneuropathy (CIDP); neuropathy associated with connective tissue diseases; Crow-Fukase syndrome (POEMS syndrome); hereditary neuropathy such as Charcot-Marie-Tooth disease; postherpetic neuralgia; peripheral neuropathy associated with AIDS or Lyme disease; uremia; multifocal motor neuropathy; and vasculitis neuropathy.
[8] The agent according to any one of [1] to [6], wherein the peripheral neuropathy is a diabetic neuropathy.
[9] The agent according to [8], wherein the diabetic neuropathy is painful diabetic neuropathy and/or asymptomatic diabetic neuropathy.
[10] The agent according to any one of [1] to [6], for use in prevention or treatment of pain symptoms associated with a disease having a peripheral neuropathy or an astrocytic damage.
[11] The agent according to [10], wherein the disease having a peripheral neuropathy or an astrocytic damage is selected from the group consisting of:

a diabetic neuropathy; entrapment neuropathy such as carpal tunnel syndrome, cubital ulnar neuropathy, peroneal nerve palsy and tarsal tunnel syndrome; familial amyloid polyneuropathy; toxic neuropathy; carcinomatous neuropathy; immune-mediated neuropathy such as Guillain-Barre syndrome (GBS) and chronic inflammatory demyelinating polyneuropathy (CIDP); neuropathy associated with connective tissue diseases; Crow-Fukase syndrome (POEMS syndrome); hereditary neuropathy such as Charcot-Marie-Tooth disease; postherpetic neuralgia;
peripheral neuropathy associated with AIDS or Lyme disease; uremia; multifocal motor neuropathy; vasculitis neuropathy; neuromyelitis optica; and Alexander's disease.

[12] The agent according to [11], wherein the disease having a peripheral neuropathy or an astrocytic damage is a diabetic neuropathy or neuromyelitis optica.
[13] The agent according to [11], wherein the disease having a peripheral neuropathy or an astrocytic damage is diabetic neuropathy.
[14] The agent according to [11], wherein the disease having a peripheral neuropathy or an astrocytic damage is neuromyelitis optica.
[15] A method of preventing or treating a peripheral neuropathy, which comprises administering an effective amount of an RGMa inhibiting substance to a mammal in need thereof.
[16] The method according to [15], wherein the peripheral neuropathy is diabetic neuropathy.

[Advantageous Effects of the Invention]

In one aspect of the present invention, an agent for preventing or treating a peripheral neuropathy can be provided. In another aspect of the present invention, an agent for preventing or treating a pain symptom associated with a disease having a peripheral neuropathy or an astrocytic damage can be provided.

BRIEF DESCRIPTION OF THE DRAWINGS

Fig. 1 is a graph showing an improvement effect of repeated administration of r116A3 on mechanical hyperalgesia in a streptozotocin (STZ)-induced rat model of diabetic neuropathy.
Fig. 2 is a graph showing an improvement effect of repeated administration of r116A3 on lowered motor nerve conduction velocity in an STZ-induced rat model of diabetic neuropathy.
Fig. 3 is a graph showing an effect of repeated administration of r116A3 on the percentage of immunostaining-positive area for glial fibrillary acidic protein (GFAP) in a spinal cord in an STZ-induced rat model of diabetic neuropathy.
Fig. 4 is a graph showing an effect of repeated administration of r116A3 on the percentage of immunostaining-positive area for Iba1 in a spinal cord in an STZ-induced rat model of diabetic neuropathy.

DETAILED DESCRIPTION OF THE INVENTION

Embodiments of the present invention will be described below.
Unless otherwise defined herein, scientific and technical terms used herein shall have the same meanings as commonly understood by those skilled in the art. Although the meaning and scope of a term must be clear, the definition given herein shall take precedence over definitions in dictionaries or outside when the meaning is unclear. Further, unless otherwise stated, a singular term shall include the plural and vice versa. As used herein, use of "or" means "and/or" unless otherwise stated.
In general, nomenclatures used in connection with, and techniques of, cell and tissue cultures, molecular biology, immunology, microbiology, genetics, protein and nucleic acid chemistry, and hybridization described herein are those known in the art and used in common. Unless otherwise indicated, the methods and techniques of the present invention are generally performed according to conventional methods known in the art and described in various general references and in the more specific references cited and discussed herein. Enzymatic reactions and purification techniques are those commonly performed in the art or are performed according to the manufacturer's specifications as described herein. Nomenclatures used in connection with, and experimental procedures and techniques of, analytical chemistry, synthetic organic chemistry, and chemistry as medicinal chemistry and medicine described herein are those known in the art and used in common. Standard techniques are used for chemical synthesis, chemical analysis, medicine, formulation, delivery and treatment of patients.
In order to facilitate the understanding of the present invention, the terms used herein will be described below.

[Neutralization]

The term "neutralization" as used herein refers to an action through which binding to a objective target and inhibition of any function of the target can be made. For example, an RGMa inhibiting substance refers to a substance which binds to RGMa to inhibit the biological activity of RGMa.

[Epitope]

As used herein, the term "epitope" includes a polypeptide determinant that can specifically bind to an immunoglobulin or a T-cell receptor. In some embodiments, an epitope can include a chemically active group on the surface of the molecule (for example, an amino acid, a sugar side chain, phosphoryl or sulfonyl). In some embodiments, an epitope can have particular characteristics of three-dimensional structure and/or electric charge. Epitopes refer to regions in antigens, to which antibodies bind.

[Isolated]

As used herein, the term "isolated" such as in isolated RGMa inhibiting substance (for example, antibody) means being identified, and separated and/or recovered from components in natural states. Impurities in natural states are substances that can interfere with the diagnostic or therapeutic use of the antibody, including enzymes, hormones and other proteinous or nonproteinous solutes. Generally, RGMa inhibiting substances or the like may be isolated through at least one purification step. RGMa inhibiting substances purified through at least one purification step can be referred to as "isolated RGMa inhibiting substances".

[Antibody]

As used herein, the term "antibody" refers broadly to an immunoglobulin (Ig) molecule comprising four polypeptide chains, two heavy chains (H chains) and two light chains (L chains), and substantially retaining characteristics of an Ig molecule to bind to an epitope.

[Human Antibody]

As used herein, the term "human antibody" refers to an antibody comprising light and heavy chains which are both derived from human immunoglobulins. Depending on the difference in the constant region of the heavy chain, human antibodies include IgG comprising a $\gamma$ heavy chain (including IgG1, IgG2, IgG3 and IgG4); IgM comprising a $\mu$ heavy chain; IgA comprising an $\alpha$ heavy chain (including IgAl and IgA2); IgD comprising a $\delta$ heavy chain; and IgE comprising an $\varepsilon$ heavy chain. In principle, as a light chain, human antibodies comprise either $\kappa$ or $\lambda$ chain.

[Humanized Antibody]

As used herein, the term "humanized antibody" refers to an antibody comprising variable regions comprising complementarity determining regions from antibodies derived from non-human animals and framework regions derived from human antibodies, and constant regions derived from human antibodies.

[Chimeric Antibody]

As used herein, the term "chimeric antibody" refers to an antibody in which the light chain, the heavy chain, or both comprises a non-human derived variable region and a human derived constant region.

[Monospecific Antibody]

As used herein, the term "monospecific antibody" refers to an antibody comprising a single independent antigen recognition site having a single antigen specificity. For example, a monospecific antibody that recognizes RGMa may be referred herein to as an RGMa-monospecific antibody.

[Multispecific Antibody]

As used herein, the term "multispecific antibody" refer to antibodies comprising two or more independent antigen recognition sites having two or more different antigen specificities, including bispecific antibodies having two antigen specificities and trispecific antibodies having three antigen specificities.

[Complementarity Determining Region (CDR)]

The term "complementarity determining region (CDR)" refers to a region forming an antigen binding site in a variable region of an immunoglobulin molecule, which is also called a hypervariable region, and particularly refers to a portion in which the amino acid sequence changes greatly for each immunoglobulin molecule. As CDRs, light and heavy chains each have three CDRs. Three CDRs contained in light chains may be referred to as LCDR1, LCDR2 and LCDR3, while three CDRs contained in heavy chains may be referred to as HCDR1, HCDR2 and HCDR3. For example, CDRs of an immunoglobulin molecule are assigned according to the Kabat numbering system (Kabat et al., 1987, Sequences of Proteins of Immunological Interest, US Department of Health and Human Services, NIH, USA).

[Effective Dose]

The term "effective dose" refers to a sufficient dose of an agent preventing or treating a disorder to alleviate or ameliorate severity and/or duration of the disorder or one or more symptoms thereof; to prevent progression of the disorder; to reverse the disorder; to prevent the recurrence, occurrence, onset or progression of one or more symptoms associated with the disorder; to detect the disorder; or to enhance or improve one or more preventive or therapeutic effects of another therapy (for example, a preventive or therapeutic drug).

[Percent (%) Identity of Amino Acid Sequence]

"Percent (%) identity" of the amino acid sequence of a candidate polypeptide sequence, such as a variable region, with respect to the amino acid sequence of a reference polypeptide sequence is defined as a percent of the same amino acid residues in the candidate sequence as the amino acid residues in the particular reference polypeptide sequence, which percent is obtained by arranging the sequences and introducing gaps if necessary to obtain maximal % identity, without considering any conservative substitutions as part of the sequence identity. Alignment for determination of % identity can be accomplished by using various methods within the skill of the art, for example, using a publicly available computer software, such as BLAST, BLAST-2, ALIGN, or Megalign (DNASTAR) software.

Those skilled in the art can determine appropriate parameters for sequence alignment, including algorithm needed to achieve maximal alignment over the full length of the sequence to be compared. However, for the purposes herein, values of % identity are obtained in pairwise alignment using a computer program for comparing sequences, BLAST.

When BLAST is used in comparison of amino acid sequences, the % identity of a given amino acid sequence A to a given amino acid sequence B is calculated as follows:

a fraction X/Y multiplied by 100

where X is the number of amino acid residues scored as identical in a programmed alignment of A and B by the sequence alignment program Blast, and Y is the total number of amino acid residues in B. It will be understood that when the lengths of amino acid sequences A and B are different, the % identities of A to B and of B to A are different. Unless stated otherwise, all % identity values herein are obtained using a computer program BLAST, as described in the immediately preceding paragraph.

The present invention is described in more detail below.

In one embodiment, the present invention provides an agent for preventing or treating a peripheral neuropathy, which comprises an RGM inhibitor, especially an RGMa inhibiting substance, as a novel use. In another embodiment, the present invention provides an agent for preventing or treating, for example, a diabetic neuropathy, which comprises an RGMa inhibiting substance. In still other embodiments, the present invention provides an agent for preventing or treating pain symptoms associated with diseases having peripheral neuropathies or astrocytic damages, which comprises an RGMa inhibiting substance.

In still other embodiments, the present invention provides a method of preventing or treating peripheral neuropathies, for example diabetic neuropathies, comprising administering a preventive or therapeutic agent containing an effective amount of an RGMa inhibiting substance to a mammal in need thereof.

<RGM Inhibiting Substance>

An RGM inhibiting substance in the present invention may be either a substance to inhibit the activity of RGM that induces peripheral neuropathies or pain symptoms associated with diseases having peripheral neuropathies or astrocytic damages described later or inhibits recovery from the diseases or conditions (hereinafter also referred to simply as "RGM activity") or a substance to inhibit the expression of RGM. RGM is one or two or more selected the group consisting of RGMa, RGMb and RGMc. Preferably, RGM is RGMa.

RGMa is identified as a protein that inhibits neurite outgrowth in central nervous systems. A human RGMa protein is biosynthesized as a precursor protein comprising 450 amino acids as shown in SEQ ID NO: 1. The signal peptide from Met 1 to Pro 47 present at the N terminus (which refers to the peptide from the first methionine residue to the 47th proline residue from the N-terminal side, and is hereafter described as above) is removed. Then, the peptide bond between Asp 168 and Pro 169 is cleaved to generate an N-terminal domain. In the C-terminal fragment from Pro 169, the peptide from Ala 425 to Cys 450 at the C terminus is removed to make Ala 424 C-terminal. Then, a GPI anchor is added to the C-terminal carboxyl group of Ala 424 to generate a C-terminal domain. The N-terminal domain (Cys 48 to Asp 168) and the C-terminal domain (Pro 169 to Ala 424) are linked by disulfide bonds to produce a mature protein, which is expressed on a cell membrane via the GPI anchor.

RGMa in the present invention may be derived from any animals. Preferably, RGMa is human RGMa. A human RGMa precursor protein may comprise the amino acid sequence shown in SEQ ID NO: 1 in Sequence Listing. A mouse RGMa precursor protein may comprise the amino acid sequence shown in SEQ ID NO: 2 in Sequence Listing, while a rat RGMa precursor protein may comprise the amino acid sequence shown in SEQ ID NO: 3 in Sequence Listing. However, removal of the C-terminal peptides results in mature proteins have the same amino acid sequence.

RGMa genes include, but are not limited to, a human RGMa gene comprising the nucleotide sequence shown in SEQ ID NO: 4. Nucleotide sequences of RGM genes derived from various organisms can be easily obtained from known databases (for example, GenBank).

An RGMa inhibiting substance in the present invention may be either a substance to inhibit (neutralize) the activity of RGMa that induces peripheral neuropathies or pain symptoms associated with diseases having peripheral neuropathies or astrocytic damages or inhibits recovery from the diseases or symptoms (hereinafter also referred to simply as "RGMa activity") or a substance to inhibit the expression of RGMa. For example, RGMa inhibiting substances in the present invention can be selected, for example, using an evaluation method described in Example 1, that is, by evaluating effects of ameliorating pains and nerve conduction disorders in an STZ-induced rat model of diabetic neuropathy.

RGMa inhibiting substances in the present invention are, for example, substances that directly inhibit the RGMa activity through binding to RGMa, or substances that indirectly inhibit the RGMa activity by interfering the binding between RGMa and its receptor, specifically including low molecular weight compounds, anti-RGMa neutralizing antibodies, functionally modified antibodies thereof, conjugated antibodies thereof and fragments thereof. In addition, substances that inhibit the RGMa activity by inhibiting the expression of RGMa are also RGMa inhibiting substances, and specifically include siRNAs (short interfering RNA), shRNAs (short hairpin RNA), and antisense oligonucleotides against RGMa genes. Among them, RGMa inhibiting substances in the present invention are preferably anti-RGMa neutralizing antibodies, functionally modified antibodies thereof, conjugated antibodies thereof, or fragments thereof, more preferably anti-RGMa neutralizing antibodies or fragments thereof, particularly preferably anti-RGMa neutralizing antibodies.

RGMa inhibiting substances in the present invention also include substances that do not act directly on RGMa but inhibit the activity of any of related molecules in signaling systems through which RGMa induces symptoms of peripheral neuropathies, for example, diabetic neuropathy.

In some embodiments of the present invention, the anti-RGMa neutralizing antibody may be an antibody that binds to RGMa to neutralize the RGMa activity. The anti-RGMa neutralizing antibody may be a polyclonal or monoclonal antibody, and is preferably a monoclonal antibody. The RGMa-neutralizing antibody of the present invention may be an RGMa-monospecific antibody or a multispecific antibody that multiply recognizes RGMa and other antigens, and preferably is an RGMa-monospecific antibody.

Specifically, the epitopes in human RGMa are preferably one or more of SEQ ID NO: 16 (amino acid numbers 47 to 69 in SEQ ID NO: 1), SEQ ID NO: 36 (amino acid numbers 298 to 311 in SEQ ID NO: 1), SEQ ID NO: 37 (amino acid numbers 322 to 335 in SEQ ID NO: 1), SEQ ID NO: 38 (amino acid numbers 349 to 359 in SEQ ID NO: 1), and SEQ ID NO: 39 (amino acid numbers 367 to 377 in SEQ ID NO: 1), more preferably a combination of SEQ ID NOS: 36 and 37, particularly preferably a combination of SEQ ID NOS: 36, 37 and 39.

The anti-RGMa neutralizing antibodies of the present invention include polyclonal or monoclonal antibodies obtained by immunizing mammals such as mice with RGMa proteins or fragments thereof (for example, epitope fragments described above) as antigens; chimeric antibodies and humanized antibodies produced by gene recombination technology; and human antibodies produced, for example, by transgenic animals producing human antibodies. When the antibody of the present invention is administered to human as a medicine, the antibody is desirably a humanized antibody or a human antibody from the viewpoint of side effects.

Specific examples of the anti-RGMa neutralizing antibody of the present invention include the following antibodies (a1) to (12), which can be produced using methods described in Patent Documents 2 to 4.

The anti-RGMa neutralizing antibody of the present invention is selected from:

(a1) anti-RGMa neutralizing antibodies comprising
a light chain variable region comprising an LCDR1 comprising the amino acid sequence represented by SEQ ID NO: 5, an LCDR2 comprising the amino acid sequence represented by SEQ ID NO: 6 and an LCDR3 comprising the amino acid sequence represented by SEQ ID NO: 7, and
a heavy chain variable region comprising an HCDR1 comprising the amino acid sequence represented by SEQ ID NO: 8, an HCDR2 comprising the amino acid sequence represented by SEQ ID NO: 9 and an HCDR3 comprising the amino acid sequence represented by SEQ ID NO: 10
(this anti-RGMa neutralizing antibodies also includes antibodies whose epitopes are SEQ ID NOS: 36, 37 and 39);
(b1) anti-RGMa neutralizing antibodies comprising
a light chain variable region comprising an LCDR1 comprising the amino acid sequence represented by SEQ ID NO: 11, an LCDR2 comprising the amino acid sequence represented by SEQ ID NO: 12 and an LCDR3 comprising the amino acid sequence represented by SEQ ID NO: 13, and
a heavy chain variable region comprising an HCDR1 comprising the amino acid sequence represented by SEQ ID NO: 14, an HCDR2 comprising the amino acid sequence represented by SEQ ID NO: 15 and an HCDR3 comprising an amino acid sequence comprising SFG
(this anti-RGMa neutralizing antibodies also includes antibodies whose epitopes are SEQ ID NOS: 36, 37 and 38);
(c1) anti-RGMa neutralizing antibodies comprising

a light chain variable region comprising an LCDR1 comprising the amino acid sequence represented by SEQ ID NO: 17, an LCDR2 comprising the amino acid sequence represented by SEQ ID NO: 18 and an LCDR3 comprising the amino acid sequence represented by SEQ ID NO: 19, and
a heavy chain variable region comprising an HCDR1 comprising the amino acid sequence represented by SEQ ID NO: 20, an HCDR2 comprising the amino acid sequence represented by SEQ ID NO: 21 and an HCDR3 comprising the amino acid sequence represented by SEQ ID NO: 22;
(d1) anti-RGMa neutralizing antibodies comprising
a light chain variable region comprising an LCDR1 comprising the amino acid sequence represented by SEQ ID NO: 23, an LCDR2 comprising the amino acid sequence represented by SEQ ID NO: 24 and an LCDR3 comprising the amino acid sequence represented by SEQ ID NO: 25, and
a heavy chain variable region comprising an HCDR1 comprising the amino acid sequence represented by SEQ ID NO: 26, an HCDR2 comprising the amino acid sequence represented by SEQ ID NO: 27 and an HCDR3 comprising the amino acid sequence represented by SEQ ID NO: 28;
(e1) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 31, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16);
(f1) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 35, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16);
(g1) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16);
(h1) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 41, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16);
(i1) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 42, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16);
(j1) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 43, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16);
(k1) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 44, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16); and
(11) anti-RGMa neutralizing antibodies comprising
a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and
a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34
(this anti-RGMa neutralizing antibodies also includes an antibody whose epitope is SEQ ID NO: 16).

More preferably, the anti-RGMa neutralizing antibody of the present invention is selected from the following (a2) to (12):

(a2) anti-RGMa neutralizing antibodies comprising
a light chain variable region comprising an LCDR1 consisting of the amino acid sequence represented by SEQ ID NO: 5, an LCDR2 consisting of the amino acid sequence represented by SEQ ID NO: 6 and an LCDR3 consisting of the amino acid sequence represented by SEQ ID NO: 7, and
a heavy chain variable region comprising an HCDR1 consisting of the amino acid sequence represented by SEQ ID NO: 8, an HCDR2 consisting of the amino acid sequence represented by SEQ ID NO: 9 and an HCDR3 consisting of the amino acid sequence represented by SEQ ID NO: 10
(this anti-RGMa neutralizing antibodies also includes antibodies whose epitopes are SEQ ID NOS: 36, 37 and 39);
(b2) anti-RGMa neutralizing antibodies comprising
a light chain variable region comprising an LCDR1 consisting of the amino acid sequence represented by SEQ ID NO: 11, an LCDR2 consisting of the amino acid sequence represented by SEQ ID NO: 12 and an LCDR3 consisting of the amino acid sequence represented by SEQ ID NO: 13, and
a heavy chain variable region comprising an HCDR1 consisting of the amino acid sequence represented by SEQ ID NO: 14, an HCDR2 consisting of the amino acid sequence represented by SEQ ID NO: 15 and an HCDR3 consisting of the amino acid sequence of SFG
(this anti-RGMa neutralizing antibodies also includes antibodies whose epitopes are SEQ ID NOS: 36, 37 and 38);
(c2) anti-RGMa neutralizing antibodies comprising
a light chain variable region comprising an LCDR1 consisting of the amino acid sequence represented by SEQ ID NO: 17, an LCDR2 consisting of the amino acid sequence represented by SEQ ID NO: 18 and an LCDR3 consisting of the amino acid sequence represented by SEQ ID NO: 19, and
a heavy chain variable region comprising an HCDR1 consisting of the amino acid sequence represented by SEQ ID NO: 20, an HCDR2 consisting of the amino acid sequence represented by SEQ ID NO: 21 and an HCDR3 consisting of the amino acid sequence represented by SEQ ID NO: 22;
(d2) anti-RGMa neutralizing antibodies comprising
a light chain variable region comprising an LCDR1 consisting of the amino acid sequence represented by SEQ ID NO: 23, an LCDR2 consisting of the amino acid sequence represented by SEQ ID NO: 24 and an LCDR3 consisting of the amino acid sequence represented by SEQ ID NO: 25, and
a heavy chain variable region comprising an HCDR1 consisting of the amino acid sequence represented by SEQ ID NO: 26, an HCDR2 consisting of the amino acid sequence represented by SEQ ID NO: 27 and an HCDR3 consisting of the amino acid sequence represented by SEQ ID NO: 28;
(e2) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 31, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16);
(f2) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 35, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16);
(g2) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 40, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16);
(h2) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 41, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16);
(i2) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 42, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16);
(j2) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 43, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16);
(k2) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 44, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16); and
(12) anti-RGMa neutralizing antibodies comprising
a light chain variable region comprising an LCDR1 consisting of the amino acid sequence represented by SEQ ID NO: 29, an LCDR2 consisting of the amino acid sequence represented by SEQ ID NO: 30 and an LCDR3 consisting of the amino acid sequence represented by SEQ ID NO: 45, and
a heavy chain variable region comprising an HCDR1 consisting of the amino acid sequence represented by SEQ ID NO: 32, an HCDR2 consisting of the amino acid sequence represented by SEQ ID NO: 33 and an HCDR3 consisting of the amino acid sequence represented by SEQ ID NO: 34
(this anti-RGMa neutralizing antibodies also includes an antibody whose epitope is SEQ ID NO: 16).

Among them, the antibodies described in (a2) are particularly preferable.

The anti-RGMa neutralizing antibodies of the present invention can be produced using production methods that are conventionally and commonly used. Antigens may be directly used for immunization, or may be used as a complex with a carrier protein. For preparing a complex of an antigen and a carrier protein, condensing agents such as glutaraldehyde, carbodiimides and maleimide active esters can be used. Examples of the carrier protein include bovine serum albumin, thyroglobulin, hemocyanin, and KLH.

Examples of the mammal to be immunized include mice, rats, hamsters, guinea pigs, rabbits, cats, dogs, pigs, goats, horses and cattle. Examples of the inoculation method include subcutaneous, intramuscular and intraperitoneal administrations. When administered, antigens may be administered in mixture with complete or incomplete Freund's adjuvant, and are usually administered once every 2 to 5 weeks. Antibody-producing cells obtained from the spleen or lymph nodes of the immunized animals are fused with myeloma cells and isolated as hybridomas. As the myeloma cells, those derived from mammals such as mouse, rat, and human are used.

The polyclonal antibodies can be obtained, for example, by immunizing the mammals as described above with the antigens as described above in combination with Freund's adjuvant as necessary, and obtaining the polyclonal antibodies from sera derived from the immunized animals.

The monoclonal antibodies can be obtained, for example, using methods described in "Current Protocols in Molecular Biology" (John Wiley & Sons (1987)), Antibodies: A Laboratory Manual, Ed. Harlow and David Lane, Cold Spring Harbor Laboratory (1988). The "hybridomas" secreting monoclonal antibodies can be prepared according to the method of Kohler and Milstein et al. (Nature, 256, 495, 1975) or a modified method based on it. Specifically, the monoclonal antibodies can be obtained as described below. The antigen as described above is used as an immunogen, and the immunogen is injected or transplanted one or several times in combination with Freund's adjuvant, as necessary, to a mammal as described above subcutaneously, intramuscularly, intravenously, into a footpad or intraperitoneally for immunization. Typically, the immunization is performed 1 to 4 times every 1 to 14 days from the initial immunization, and after about 1 to 5 days from the final immunization, antibody-producing cells are obtained from the immunized mammal.

The hybridoma is prepared by fusing the antibody-producing cell and a myeloma cell without ability to produce an autoantibody, which is derived from a mammal, preferably mouse, rat or human, using a fusion promoter as necessary.

Examples of the myeloma cell which can be used in the cell fusion include mouse-derived myelomas such as P3/X63-AG8.653 (653), P3/NSI/1-Ag4-1 (NS-1), P3/X63-Ag8.U1 (P3U1), SP2/0-Ag14 (Sp2/O, Sp2), PAI, F0 and BW5147; rat-derived myelomas such as 210RCY3-Ag.2.3.; and human-derived myelomas such as U-266AR1, GM1500-6TG-A1-2, UC729-6, CEM-AGR, D1R11 and CEM-T15.

Examples of the fusion promoter include polyethylene glycols. The cell fusion can usually be performed by allowing the antibody-producing cells and the myeloma cells to react at a cell number ratio usually from about 1: 1 to 10: 1, using polyethylene glycols (with an average molecular weight from 1000 to 4000) with a concentration from about 20 to 50%, at a temperature from 20 to 40°C, preferably from 30 to 37°C, for about 1 to 10 minutes.

Screening of hybridoma clones producing the monoclonal antibodies can be carried out by culturing the hybridomas, for example, in a microtiter plate and assaying the culture supernatants in the wells for the reactivity against an immunogen by an immunochemical method such as ELISA.

In the screening of antibody-producing hybridomas, whether the antibody inhibits the RGMa activity in the present invention is also determined in addition to the binding assay with an RGMa protein. The screening methods allow for selection of the anti-RGMa neutralizing antibody of the present invention.

Clones can be further obtained from the wells containing hybridomas producing the desired antibodies by cloning with limiting dilution. Hybridomas are usually selected and grown in an animal cell culture medium containing 10 to 20% fetal bovine serum, supplemented with HAT (hypoxanthine, aminopterin and thymidine).

The monoclonal antibodies can be produced from the hybridomas by culturing the hybridomas in vitro or growing them in vivo, for example, in ascitic fluids of mammals, such as mice and rats, and isolating the monoclonal antibodies from the resulting culture supernatants or the ascitic fluids of the mammals.

When cultured in vitro, according to various conditions such as the characteristics of and the method for culturing the cell species to be cultured, hybridomas can be grown, maintained, and stored, while using nutrient media suitable for producing monoclonal antibodies in the culture supernatant. As the nutrient medium, for example, a known nutrient medium or a nutrient medium prepared from a basal medium can be used.

Examples of the basal media include low-calcium media such as Ham's F12 medium, MCDB 153 medium and low-calcium MEM medium, and high-calcium media such as MCDB 104 medium, MEM medium, D-MEM medium, RPMI 1640 medium, ASF 104 medium and RD medium. The basal medium can contain according to the purpose, for example, sera, hormones, cytokines and/or various inorganic or organic substances.

The monoclonal antibodies can be isolated and purified by, for example, subjecting the above-described culture supernatant or ascitic fluid to saturated ammonium sulfate, euglobulin precipitation, caproic acid treatment, caprylic acid treatment, an ion exchange chromatography (such as DEAE or DE52) or an affinity column chromatography such as anti-immunoglobulin column or protein A column chromatography. Specifically, the monoclonal antibodies may be purified by any method known as immunoglobulin purification method, and the purification can be easily achieved by means such as ammonium sulfate fractionation treatment, PEG fractionation treatment, ethanol fractionation treatment, use of anion exchangers and further affinity chromatography methods using RGMa proteins.

The monoclonal antibodies can also be obtained by a phage display method. In a phage display method, phages selected from a phage antibody library are screened using a desired immunogen, and phages having a desired binding ability to the immunogen are selected. Next, the antibody-corresponding sequence contained in the phage is isolated or sequenced. Based on the information from the isolated sequence or the sequencing, an expression vector comprising a nucleic acid molecule encoding the antibody or antigen binding domain is constructed. The expression vector is then transfected into a cell line, and the cell line can be cultured to produce a monoclonal antibody. When a human antibody library is used as the phage antibody library, a human antibody having a desired binding ability can be produced.

For example, a nucleic acid molecule encoding an anti-RGM antibody or a fragment thereof can be obtained by the following method. First, total RNA is prepared from a cell such as hybridoma using a commercially available RNA extraction kit, and subsequently cDNA is synthesized by a reverse transcriptase using random primers and the like. Next, by a PCR method using, as primers, oligonucleotides having conserved gene sequences in variable regions in heavy and light chain in a known human antibody, cDNAs encoding the antibody are amplified. Sequences encoding the constant regions can be obtained by amplification of known sequences by a PCR method. The nucleotide sequence of the DNA can be sequenced by a conventional method, for example, by incorporating it into a plasmid for sequencing.

Alternatively, a DNA encoding the monoclonal antibody of the present invention can also be obtained by chemically synthesizing sequences of the variable regions or parts thereof and joining them to sequences comprising the constant regions.

The nucleic acid molecule may encode all of the constant regions and the variable regions of heavy and light chains, or may encode only the variable regions of heavy and light chains. In the case of encoding all of the constant regions and the variable regions, the nucleotide sequences of the constant regions of heavy and light chains are preferably those described in Nucleic Acids Research vol.14, p1779, 1986; The Journal of Biological Chemistry vol.257, p1516, 1982; and Cell vol.22, p197, 1980.

The functionally modified antibodies are prepared by the following methods. For example, half-life in blood can be extended by using a Fc region variant with increased binding to FcRn, one of Fc receptors (Hashiguchi Shuhei et al., The Journal of Japanese Biochemical Society, Vol.82 (8), p710 (2010)). These functionally modified antibodies can be produced by genetic engineering techniques.

Examples of the conjugated antibodies include anti-RGMa neutralizing antibodies that are bound chemically or by genetic engineering to function molecules other than the present anti-RGMa neutralizing antibody, such as nonpeptidic polymers such as polyethylene glycol (PEG), radioactive substances, toxins, low molecular weight compounds, cytokines, growth factors (such as TGF-β, NGF, neurotrophin), albumin, enzymes, and other antibodies.

When PEG is bound as the functional molecule, PEG can be used having a non-limiting molecular weight from 2,000 to 100,000 Da, more preferably from 10,000 to 50,000 Da, which may be linear or branched. By using an NHS active group or the like, PEG can be bound, for example, to the N-terminal amino group in amino acids of the RGMa inhibiting substance.

When a radioactive substance is used as the functional molecule, examples include $^{131}I$, $^{125}I$, $^{90}Y$, $^{64}Cu$, $^{99}TC$, $^{77}Lu$, and $^{211}At$. The radioactive substance can be directly bound to the RGMa inhibiting substance by a chloramine-T method or the like.

When a toxin is used as the functional molecule, examples include bacterial toxins (such as diphtheria toxin), phytotoxins (such as ricin), small toxins (such as geldanamycin), maytansinoids and calicheamicin are used.

When a low molecular weight compound is used as the functional molecule, examples include daunomycin, doxorubicin, methotrexate, mitomycin, neocarzinostatin, vindesine and fluorescent dyes such as FITC.

When an enzyme is used as the functional molecule, examples include luciferases (such as firefly luciferases and bacterial luciferases; US Patent No. 4,737,456), malate dehydrogenases, ureases, peroxidases (such as horseradish peroxidase (HRPO)), alkaline phosphatases, β-galactosidases, glucoamylases, lysozymes, saccharide oxidases (such as glucose oxidase, galactose oxidase and glucose-6-phosphate dehydrogenase), heterocyclic oxidases (such as uricase and xanthine oxidase), lactoperoxidases, and microperoxidases.

Examples of linkers used for chemically bonding the toxin, low molecular weight compound or enzyme include divalent radicals (such as alkylene, arylene, and heteroarylene), linkers represented by $-(CR_2)_nO(CR_2)_n-$ (wherein R is any substituent, and n is a positive integer), alkoxy repeating units (such as polyethylene oxy, PEG, and polymethyleneoxy), alkylamino repeating units (such as polyethyleneamino, and Jeffamine™), and diacid esters and amides (including succinate, succinamide, diglycolate, malonate and capramide). Chemical modification methods for binding functional molecules have already been established in this field (D. J. King., Applications and Engineering of Monoclonal antibodies., 1998 T.J. International Ltd, Monoclonal Antibody-Based Therapy of Cancer., 1998 Marcel Dekker Inc; Chari et al., Cancer Res., 1992 Vol.152: 127; Liu et al., Proc Natl Acad Sci USA., 1996 Vol 93: 8681).

In embodiments of the present invention, "fragments" of antibodies means partial regions having antigen binding properties derived from the antibodies as described above. Specific examples of the fragments include F(ab')$_2$, Fab', Fab, Fv (variable fragment of antibody), disulfide-linked Fv, single-chain antibodies (scFv), and polymers thereof. Examples of the fragments also include conjugated fragments that are bound chemically or by genetic engineering to function molecules other than the present anti-RGMa neutralizing antibody, such as nonpeptidic polymers such as polyethylene glycol (PEG), radioactive substances, toxins, low molecular weight compounds, cytokines, growth factors (such as TGF-β, NGF, and neurotrophin), albumin, enzymes, and other antibodies.

The terms "F(ab')$_2$" and "Fab" means antibody fragments produced by treating an immunoglobulin with a protease such as pepsin or papain, or produced by digestion at the upstream or downstream side of the disulfide bond existing between two heavy chains in the hinge region. For example, IgG can be treated with papain to be cleaved at the upstream side of the disulfide bond existing between the two heavy chains in the hinge region and produce two homologous antibody fragments each comprising a light chain comprising a VL (light chain variable region) and a CL (light chain constant region) and a heavy chain fragment comprising a VH (heavy chain variable region) and a CHγ1 (γ1 region in the heavy chain constant region) in which the light chain and the heavy chain fragment are linked to each other via a disulfide bond at the C-terminal domain. The two homologous antibody fragments are referred to as Fab. IgG can be treated with Pepsin to be cleaved cleavage at the downstream side of the disulfide bond existing between the two heavy chains in the hinge region and produce an antibody fragment that is slightly larger than the two Fabs linked to each other at the hinge region. This antibody fragment is referred to as F(ab')$_2$.

In a preferred embodiment of the present invention, the anti-RGMa neutralizing antibody is a chimeric antibody. Examples of the "chimeric antibody" include those in which the variable regions are derived from immunoglobulins from non-human animals (such as mouse, rat, hamster and chicken) and the constant regions are derived from human immunoglobulins. The chimeric antibody can be prepared, for example, by immunizing a mouse with the antigen, cleaving out a variable region that binds to the antigen from the gene encoding the mouse monoclonal antibody, and combining the variable region with a constant region of an antibody derived from human bone marrow. The constant region derived from a human immunoglobulin has a unique amino acid sequence depending on the isotype, such as IgG (IgG1, IgG2, IgG3 or IgG4), IgM, IgA (IgA1 or IgA2), IgD or IgE. The constant region of the recombinant chimeric antibody according to the present invention may be a constant region of a human immunoglobulin belonging to any of the isotypes. The constant region is preferably a constant region of human IgG. The chimeric antibody gene thus prepared can be used to prepare an expression vector. A host cell is transformed with the expression vector to obtain a transformed cell that produces the chimeric antibody. Subsequently, the transformed cell is cultured to obtain the desired chimeric antibody from the culture supernatant.

In another preferred embodiment of the present invention, the anti-RGMa neutralizing antibody is a humanized antibody. The "humanized antibody" according to the present invention is an antibody obtained by grafting only the DNA sequence of the antigen binding site (CDRs; complementarity determining regions) of an antibody derived from a non-human animal such as mouse to a human antibody gene (CDR grafting). The humanized antibody can be prepared according to the methods described in, for example, Japanese Unexamined Patent Application Publication No. 1992-506458 and Japanese Patent No. 2912618. Specifically, the humanized antibody comprises CDRs partly or wholly derived from monoclonal antibodies from non-human mammals (such as mouse, rat and hamster); framework regions derived from variable regions of human immunoglobulins; and constant regions derived from human immunoglobulins.

The humanized antibody according to the present invention can be produced, for example, as described below. Needless to say, however, the production method is not limited thereto.

For example, a recombinant humanized antibody derived from a mouse monoclonal antibody can be produced by genetic engineering with reference to Japanese Unexamined Patent Application Publication No. 1992-506458 and Japanese Unexamined Patent Application Publication No. 1987-296890. Specifically, DNA encoding the part of CDRs of a mouse heavy chain and DNA encoding the part of CDRs of a mouse light chain are isolated from hybridomas producing a mouse monoclonal antibody. Further, a human heavy chain gene encoding the whole region other than CDRs of the human heavy chain and a human light chain gene encoding the whole region other than CDRs of the human light chain are isolated from a human immunoglobulin gene.

The isolated DNA encoding the part of CDRs of the mouse heavy chain is grafted to the human heavy chain gene, and the product is introduced into an appropriate expression vector so that it is expressible. Similarly, the DNA encoding the part of CDRs of the mouse light chain is grafted to the human light chain gene, and the product is introduced into another appropriate expression vector so that it is expressible. Alternatively, the human heavy and light chain genes to which mouse CDRs are grafted may be introduced into the same expression vector so that they are expressible. A host cell is transformed with the expression vector thus prepared to obtain a transformed cell producing the humanized antibody. Subsequently, the transformed cell is cultured to obtain the desired humanized antibody from the culture supernatant.

In another preferred embodiment of the present invention, the anti-RGMa neutralizing antibody is a human antibody. The term "human antibody" refers to an antibody in which the entire region including heavy chain variable regions and heavy chain constant regions and light chain variable regions and light chain constant regions constituting the immunoglobulin are derived from genes encoding human immunoglobulins. Human antibodies can be prepared by introducing human antibody genes into mice. Human antibodies can be produced in the same manner as the above-described method for preparing polyclonal antibodies or monoclonal antibodies. Specifically, for example, the method comprises introducing at least human immunoglobulin genes into gene loci of a non-human mammal such as mouse to prepare a transgenic animal and immunizing the transgenic animal with an antigen.

For example, a transgenic mouse that produces a human antibody can be prepared according to the methods described, for example, in Nature Genetics, Vol.7, p.13-21, 1994; Nature Genetics, Vol.15, p.146-156, 1997; Japanese Unexamined Patent Application Publication Nos. 1992-504365 and 1995-509137; WO 94/25585; Nature, Vol.368, p.856-859, 1994; and Japanese Translated Unexamined Application Publication No. 1994-500233. More specifically, for example, HuMab® Mouse (Medarex, Princeton NJ), KMTM mouse (Kirin Pharma Company, Japan), and KM (FCyRIIb-KO) mouse are used.

In specific embodiments of the present invention, the anti-RGMa neutralizing antibody has CDRs comprising specific amino acid sequences in the heavy chain variable region, and has CDRs comprising specific amino acid sequences in the light chain variable region (the antibodies (a1) to (12) described above).

The amino acid sequence of the anti-RGMa neutralizing antibody may have substitution, deletion, addition or insertion of one or several (1 to 20, 1 to 10, 1 to 5, 1 to 3, or 1 to 2) amino acids as long as the antibody of the present invention maintains the characteristics of having an ability to bind to RGMa and inhibiting (neutralizing) the activity of RGMa. The substitution, deletion, or addition may be introduced in CDRs. However, it is preferably introduced in a region other than CDR. The amino acid substitution is preferably conservative substitution in order to maintain the characteristics of the present invention.

When the amino acid sequence of the antibody of the present invention has a substitution, deletion, or the like, for example the amino acid sequence after the modification of the heavy chain variable region is 90% or more (more preferably 95%, 96%, 97%, 98%, 99% or more,) identity to the amino acid sequence before the modification, and the amino acid sequence after the modification of the light chain variable region is 90% or more (more preferably 95%, 96%, 97%, 98%, 99% or more,) identity to the amino acid sequence before the modification.

The term "siRNA" refers to a short double strand RNA capable of inhibiting the expression of a target gene (a RGMa gene in the present invention). The nucleotide sequence and the length (base length) are not particularly limited as long as the function as an siRNA to inhibit the RGMa activity in the present invention is maintained. The base length is preferably less than about 30 bases, more preferably about 19 to 27 bases, still more preferably about 21 to 25 bases. The term "shRNA" refers to a single strand RNA partially comprising a palindromic nucleotide sequence to form a double-stranded structure in the molecule, resulting in an about 20 base pairs or more molecule having a 3' overhang and a short hairpin structure. Such an shRNA can be introduced into a cell and then degraded into a length of about 20 bases (typically, for example, 21, 22, or 23 bases) in the cell to inhibit the expression of a target gene similarly to siRNA. The siRNA and shRNA in the present invention may be in any form as long as they can inhibit the expression of the RGMa gene.

An artificial siRNA or shRNA can be chemically synthesized. Antisense and sense RNAs can also be synthesized in vitro from DNA templates using, for example, a T7 RNA polymerase and a T7 promoter. The antisense oligonucleotide may be a nucleotide that is complementary or hybridizes to a consecutive nucleotide sequence having a length from 5 to 100 in the DNA sequence of an RGMa gene, and may be DNA or RNA. The antisense oligonucleotide may be modified without impairing its function. The antisense oligonucleotide can be synthesized by a conventional method. For example, the antisense oligonucleotide can be easily synthesized with a commercially available DNA synthesizer.

A preferred sequence can be selected by a common selection method, and the validation as the siRNA or shRNA according to the present invention can be made by evaluating inhibition of the expression of a functional RGMa.

In embodiments of the present invention, the peripheral neuropathy is, for example, a diabetic neuropathy, entrapment neuropathy such as carpal tunnel syndrome, cubital ulnar neuropathy, peroneal nerve palsy and tarsal tunnel syndrome, familial amyloid polyneuropathy, toxic neuropathy, carcinomatous neuropathy, immune-mediated neuropathy such as Guillain-Barre syndrome (GBS) and chronic inflammatory demyelinating polyneuropathy (CIDP), neuropathy associated with connective tissue diseases, Crow-Fukase syndrome (POEMS syndrome), hereditary neuropathy such as Charcot-Marie-Tooth disease, postherpetic neuralgia, peripheral neuropathy associated with AIDS or Lyme disease, uremia, multifocal motor neuropathy or vasculitis neuropathy.

In other embodiments of the present invention, the diseases having an astrocytic damage include diseases exhibiting an astrocytic damage or astrocytic hypofunction, specifically includes neuromyelitis optica, and Alexander's disease.

In these embodiments, preferred peripheral neuropathies include diabetic neuropathy. Preferred diseases having astrocytic damages include neuromyelitis optica.

In other embodiments of the present invention, the pain symptoms associated with diseases having peripheral neuropathies or astrocytic damages include symptoms caused by peripheral nerve injuries, or astrocytic damages or hypofunctions in the central nervous system. In the present invention, diseases having peripheral neuropathies or astrocytic damages which cause pain symptoms are synonyms of the diseases having peripheral neuropathies or astrocytic damages described above.

For example, with respect to diseases having peripheral neuropathies causing such pain symptoms, as shown in Example 1 (1-6) described later, an RGMa inhibiting substance, for example, an anti-RGMa antibody improves the nerve conduction velocity, which suggests that RGMa inhibiting substances treat diseases having peripheral neuropathies. In addition, an RGMa inhibiting substance also reduces pains, which suggests that RGMa inhibiting substances show analgesic effects against pains associated with diseases having peripheral neuropathies.

As a mechanism of action possibly causing pain symptoms associated with diseases having astrocytic damages, astrocytic damages or hypofunctions would cause a decrease in the expression of glutamate transporter in central nerves, resulting in enhancement of the action of glutamate, and supersensitization or excitement of the nerves (for example, Neuron 67, 834-846, Sep.9, 2010). The pain symptoms include a symptom associated with the onset of the diseases, such as numbness, ache or hypesthesia, due to astrocytic damages.

Thus, as shown in Example 1 (1-6) described later, in a STZ-induced rat model of diabetic neuropathy, in which a peripheral neuropathy and an astrocytic damage are induced, which results in development of pains, an RGMa inhibiting substance, for example, an anti-RGMa antibody shows an analgesic effect against the pain. On the other hand, an RGMa inhibiting substance, for example, an anti-RGMa neutralizing antibody has no effect on decrease in microglia, but shows an effect of suppressing the decrease in astrocytes, which suggests that an RGMa inhibiting substance, for example, an anti-RGMa neutralizing antibody shows an analgesic effect against pains associated with diseases having astrocytic damages or hypofunctions. Thus, from the experimental results in Example 1, RGMa inhibiting substances, preferably anti-RGMa neutralizing antibodies, are expected to show an effect on pain symptoms associated with one or both of the diseases having peripheral neuropathies or astrocytic damages. In Non-patent Document 10, experiments on astrocytic damages in neuromyelitis optica have been performed. However, since this experimental model is an animal model not available for evaluation of ache, the relationship between diseases having astrocytic damages and pain symptoms is unknown from this document. Thus, the relationship between diseases having astrocytic damages and pain symptoms is a novel finding obtained in Example 1 herein.

In preferred embodiments of the present invention, for example, diabetic neuropathies, which are most common complications in diabetic patients, are caused by known developmental risk factors such as poorly-controlled blood glucose, long-term duration of diabetes mellitus, hypertension, and dyslipidemia, but have not yet been identified for the pathogenic mechanism. Diabetic neuropathies are divided into distal symmetric polyneuropathies and local mononeuropathies. In particular, the former is considered as core symptoms of diabetic neuropathies and include sensory neuropathies, motor neuropathies, and autonomic neuropathies.

There are no specific symptoms or tests for diagnosis of diabetic neuropathies. Thus, comprehensive judgment is made through careful auscultation for neurological symptoms in patients and neurological examinations such as pain sensation, vibration sensation, haptic sensation, and tendon reflex test.

Major symptoms of diabetic neuropathies include sensory neuropathies, motor neuropathies, and autonomic neuropathies. Sensory neuropathies include painful neuropathies with marked paralgesia and asymptomatic neuropathies without spontaneous paresthesia. Sensory neuropathies cause numbness and pain at the end of a lower limb early in their development, and thereafter, marked hypesthesia appears as the symptoms progress. Furthermore, progression of the symptoms also leads to appearance of symptoms such as numbness, pain and hypesthesia not only at the end of lower limbs but also at the end of upper limbs. Sensory neuropathies not only lead to lowering of QOL in patients, but also may lead to, after progression of the symptoms and appearance of hypesthesia, higher risks of foot gangrene and Charcot's joint, which may further lead to quadruple amputation and deterioration of vital prognosis. Therefore, for diabetic neuropathies, therapeutic interventions are critically needed from the early stage of development. On the other hand, motor neuropathies are characterized by, in particular, symptoms such as lower limb muscle weakness, muscle atrophy, and foot deformity. Though typically not noticeable enough to affect daily activities, these symptoms come to affect loaded walking, such as maintaining of balance, rising and falling on hills and stairs, and fast walking as the symptoms progress.

The agent for preventing or treating diabetic neuropathies according to the present invention can be used to ameliorate symptoms found in the test or to prevent or treat a symptom selected from the symptoms.

Among those showing the symptoms, diabetic neuropathies to be prevented or treated by the agent for preventing or treating diabetic neuropathies according to the present invention preferably include painful diabetic neuropathies, and asymptomatic diabetic neuropathies, more preferably painful diabetic neuropathies.

As used herein, the term "treat" includes any treatment of diseases in mammals, especially human, and includes inhibiting symptoms of the diseases, or inhibiting their progression or eliminating the diseases or symptoms, and alleviating symptoms of the disease, or inducing regression of the diseases or symptoms or delay of the progression of the symptoms. The term "treat" of the present invention means directly treating diabetic neuropathies, not treating diabetic neuropathies based on therapies for diabetes mellitus, such as hypoglycemic action. In other embodiments of the present invention, the term "treat" means therapeutic, nerve-regenerating or nerve-protecting, local or systemic treatment.

The term "prevent" includes preventing onset of the above-described diseases in mammals, especially human.

The agent for preventing or treating peripheral neuropathies, or pain symptoms associated with diseases having peripheral neuropathies or astrocytic damages according to the present invention is usually administered systemically or locally in and oral or parenteral from.

The agent for preventing or treating peripheral neuropathies, or pain symptoms associated with diseases having peripheral neuropathies or astrocytic damages according to the present invention can be formulated into a pharmaceutical composition comprising an RGMa inhibiting substance as an active ingredient, and a pharmaceutically acceptable carrier or additive as appropriate. Specifically, the agent can be formulated into oral agents such as tablets, coated tablets, pills, powders, granules, capsules, solutions, suspensions, and emulsions; or parenteral agents such as injections, infusions, suppositories, ointments, and patches. The content ratio of the carrier or additive may be set as appropriate according to the ranges commonly used in the pharmaceutical field. The carrier or additive that can be added is not particularly limited, and includes various carriers such as water, physiological saline, other aqueous solvents, aqueous or oily bases; and various additives such as excipients, binders, pH adjusters, disintegrants, absorption promoters, lubricants, colorants, flavoring agents, and perfumes.

When the RGMa inhibiting substance is an anti-RGMa neutralizing antibody, a functionally modified antibody thereof, a conjugated antibody thereof or a fragment thereof, the agent is preferably formulated into an injection or infusion with a pharmaceutically acceptable carrier and administered via a parenteral route of administration, such as intravenous, intramuscular, intradermal, intraperitoneal, subcutaneous or local administration. The injection or infusion containing the anti-RGMa neutralizing antibody can be used as a solution, suspension or emulsion. Examples of the solvent include distilled water for injection, physiological saline, dextrose in water and isotonic solutions (for example, solutions of sodium chloride, potassium chloride, glycerin, mannitol, sorbitol, borate, sodium borate, and propylene glycol). The injection or infusion may further contain, for example, a stabilizer, a solubilizer, a suspending agent, an emulsifier, a soothing agent, a buffer agent, a preservative, an antiseptic, and a pH adjuster. Examples of the stabilizer include albumin, globulin, gelatin, mannitol, glucose, dextran, ethylene glycol, propylene glycol, ascorbic acid, sodium bisulfite, sodium thiosulfate, sodium EDTA, sodium citrate, and dibutyl hydroxytoluene. Examples of the solubilizer include alcohols (such as ethanol), polyalcohols (such as propylene glycol and polyethylene glycol), and nonionic surfactants (such as polysorbate80®, and HCO-50). Examples of the suspending agent include glyceryl monostearate, aluminum monostearate, methylcellulose, carboxymethylcellulose, hydroxymethylcellulose, and sodium lauryl sulfate. Examples of the emulsifier include gum arabic, sodium alginate, and tragacanth. Examples of the soothing agent include benzylalcohol, chlorobutanol, and sorbitol. Examples of the buffer agent include a phosphate buffer, acetate buffer, borate buffer, carbonate buffer, citrate buffer, and Tris buffer. Examples of the preservative include methyl parahydroxybenzoate, ethyl parahydroxybenzoate, propyl parahydroxybenzoate, butyl parahydroxybenzoate, chlorobutanol, benzylalcohol, benzalkonium chloride, sodium dehydroacetate, sodium edetate, boric acid, and sodium borate. Examples of the antiseptic include benzalkonium chloride, parahydroxybenzoic acid, and chlorobutanol. Examples of the pH adjuster include hydrochloric acid, sodium hydroxide, phosphoric acid, and acetic acid.

When the RGMa inhibiting substance is a nucleic acid (such as a nucleic acid molecule encoding an siRNA, shRNA, antisense oligonucleotide, or an anti-RGMa neutralizing antibody or a fragment thereof), it can be administered in the form of a nonviral or viral vector. When the RGMa inhibiting substance is administered in the form of a nonviral vector, for example, a method of introducing a nucleic acid molecule using a liposome (such as a liposome method, HVJ-liposome method, cationic liposome method, lipofection, or Lipofectamine method), microinjection, or a method of transferring a nucleic acid molecule with a carrier (a metal particle) into a cell using a gene gun can be used. When the RGMa inhibiting substance is administered in the form of a viral vector, for example, a viral vector such as a recombinant adenovirus or retrovirus can be used. A DNA that express the siRNA or shRNA can be introduced to a DNA virus or RNA virus such as an attenuated retrovirus, adenovirus, adeno-associated virus, herpesvirus, vaccinia virus, poxvirus, poliovirus, Sindbis virus, Sendai virus, or SV40. A cell or tissue can be infected with the recombinant virus to introduce the gene into the cell or tissue.

The formulation thus obtained can be administered to a subject in need thereof, for example, a human or another mammal (such as a rat, mouse, rabbit, sheep, pig, cattle, cat, dog, or monkey) at an effective dose to prevent or treat peripheral neuropathies, for example, diabetic neuropathies or, pain symptoms associated with diseases having peripheral neuropathies or astrocytic damages. The dosage is set as appropriate depending on, for example, purposes, severities of diseases, ages, weights, sexes, and past histories of patients, and types of active ingredients. For example, when the active ingredient is an anti-RGMa neutralizing antibody, the dosage for an average human having a weight of about 65 to 70 kg is preferably about 0.02 mg to 4000 mg per day, more preferably about 0.1 mg to 200 mg per day. The total dosage per day may be a single dose or divided doses.

The agent for preventing or treating peripheral neuropathies, or pain symptoms associated with diseases having peripheral neuropathies or astrocytic damages according to the present invention can be used in combination therapy or as a mixture with a conventional anti-pain agent.

Examples of the agent used in combination with the present invention include anti-pain agents and antidiabetic agent.

Examples of the anti-pain agents that can be used in combination therapy or as a mixture with the present invention include tricyclic antidepressants (such as amitriptyline, and imipramine), $\alpha 2\delta$ ligands (such as pregabalin), Na channel blockers (such as mexiletine), antiepileptic drugs (such as gabapentin, and carbamazepine), aldose reductase inhibitors (such as epalrestat), and serotonin noradrenaline reuptake inhibitors (SNRI, for example, duloxetine).

Examples of the antidiabetic agents that can be used in combination therapy or as a mixture with the present invention include sulfonylurea antidiabetic agents, biguanide antidiabetic agents, insulin resistance improving agent (such as pioglitazone), alpha-glucosidase inhibitors, DPP-4 inhibitors, SGLT-2 inhibitors, GLP-1 analogs, and insulin analogs.

EXAMPLES

The present invention will be described in more detail with reference to Examples, which do not limit the scope of the present invention.

[Example 1]

Studies of Effects of the Anti-RGMa Neutralizing Antibody on Pains and Motor Nerve Conduction Disorders in a STZ-Induced Rat Model of Diabetic Neuropathy

The STZ-induced rat model of diabetic neuropathy is a model for evaluation of effects on peripheral neuropathy, especially peripheral neuropathy in diabetic neuropathy. In addition, the model can be used for evaluation of effects on peripheral neuropathy, especially pain symptoms in diabetic neuropathy, as well as for evaluation of effects on pain symptoms associated with diseases having astrocytic damages.

(1-1) Preparation of STZ-Induced Rat Model of Diabetic Neuropathy

SD male rats were used for the experiments. STZ (streptozocin) (60 mg/kg) dissolved in 0.75 mM citrate buffer (pH 4.5) into a concentration of 30% was administered into a tail vein of the rat. After 3 weeks, rats achieving a blood glucose level of 300 mg/dl or more were used as a STZ experimental group (diabetes group). Rats that had the same age as the experimental group and were not administered with STZ were used as a normal group.

(1-2) Behavioral Pain Assessment

In a von Frey stimulation test, the 50% withdrawal threshold (g) observing elevation of a hind paw was determined using the up-down method (see Chaplan, S.R., Bach, F.W., Pogrel, J.W., Chung, J.M., Yaksh, T.L., Quantitative assessment of tactile allodynia in the rat paw, J. Neurosci. Methods, 53, 55-63(1994)) to evaluate the mechanical hyperalgesia.

(1-3) Measurement of Motor Nerve Conduction Velocity (MNCV)

A rat was anesthetized by continuous inhalation of isoflurane gas and held in the prone position. The MNCV was measured while maintaining the body temperature (rectal temperature) constant (37.5 to 38.5°C) with a body temperature regulator (ATB-1100, Nihon Kohden Corporation).

Needle electrodes (negative electrodes) were inserted at an ischial tuberosity site of the right sciatic nerve as a near distal stimulus location (S1), and at an ankle joint site of the right tibial nerve as a far proximal stimulus location (S2). In addition, an indifferent electrode (positive electrode) was inserted at a site about 1 cm spinal side from S1. For recording, a leading electrode (negative electrode) and a reference electrode (positive electrode) were inserted shallowly at right plantaris muscle sites. The reference electrode (positive electrode) was placed on the distal side of the negative electrode. Electrical stimuli of a single rectangular pulse (stimulation frequency: 1 Hz, duration: 0.1 msec, current: supramaximal, number of stimulation: once) were applied to S1 and S2 using an evoked potential recorder [Neuropack $\mu$ (model: MEB-9102, Nihon Kohden Corporation)], and changes in the action potential obtained with the recording electrodes were recorded. Latencies from the point of stimulation to the rising phase of the action potential for both S1 and S2 stimulations, t1 and t2 (msec), respectively, and the distance between S1 and S2, d (mm), were measured and then MNCV was calculated from the following formula:

$$MNCV = d / (t1 - t2)$$

(1-4) Grouping and Administration of Anti-RGMa Neutralizing Antibody

Three weeks after the STZ administration, animals with a blood glucose level of 300 mg/dL or less were excluded from subjects of grouping. Then, the remaining animals were grouped so that the means and variances of body weight, 50% withdrawal threshold, and MNCV were homogeneous. The STZ/control antibody-treated group, STZ/anti-RGMa neutralizing antibody-treated group, and normal/ control antibody-treated group were each comprised of ten animals.
An anti-RGMa neutralizing antibody or a control antibody (mouse IgG) was administered into the tail vein at a dose of 10 mg/kg once a week for a total of 4 times, starting from the time point of three weeks after the STZ administration.
The anti-RGMa neutralizing antibody used was r116A3, which was prepared by the method described in the reference (see Patent Document 4).

(1-5) Histopathological Evaluation with Immunostaining of GFAP and Iba1

After 28 days from the first administration of the test substance (after 51 days from the STZ administration), animals were euthanized by exsanguination while perfusing physiological saline, and fixed by perfusion with 10 vol% neutral buffered formalin. Thereafter, the spinal cord was removed and fixed by immersing it with 10 vol% neutral buffered formalin.
GFAP (staining astrocytes) and Iba1 (staining microglia) were immunostained, in order to evaluate the expressions of GFAP and Iba1 histopathologically under a light microscope.
A slide sample was photographed on a digital slide scanner Aperio (Aperio AT2, Leica Microsystems) (20x magnification). Then, the 100% image of the entire sample was extracted and converted to a JPEG image. Using an image analysis software Image-Pro premier (ver.9.3.2, Media Cybernetics), the anterior horns (ventral horns) and the posterior horns (dorsal horns) in the gray matter region were enclosed as Areas of Interest (AOIs). The stained positive areas in the SOIs were extracted and measured (size: > 1 $mm^2$), and the % stained positive areas per total areas of the SOIs were calculated. The obtained results are shown as individual values and mean $\pm$ standard error. Student's t tests were performed for the % stained areas between the non-diabetes group and the diabetes group and between the diabetes group and the diabetes + test substance-treated group.

(1-6) Results

Fig. 1 shows the effect of repeated administration of the anti-RGMa neutralizing antibody on mechanical hyperalgesia. The 50% withdrawal threshold, which had been reduced by induction of diabetes mellitus, was significantly ameliorated from week 1 after the administration of the anti-RGMa neutralizing antibody. The amelioration effect became stronger week by week, and the effect continued until at least week 4 at the final measurement point.
Fig. 2 shows the effect of repeated administration of the anti-RGMa neutralizing antibody on a motor nerve conduction disorder. The anti-RGMa neutralizing antibody also showed an amelioration effect on the motor nerve conduction velocity, which had been reduced by induction of diabetes mellitus, resulting in a significant amelioration at week 4 after the start of administration.
Fig. 3 shows the results of calculation of the % GFAP-positive area. For the anterior horn, a significant decrease in the % GFAP-positive area was observed in the diabetes group as compared with the non-diabetes group. For the anterior horn and posterior horn, a significant increase was observed in the diabetes + test substance-treated group as compared with the diabetes group.
Fig. 4 shows the results of calculation of the % Iba1-positive area. For the anterior horn and posterior horn, a significant decrease was observed in the diabetes group as compared with the non-diabetes group.

<Explanation of the Sequence Listing>

SEQ ID NO: 1: Amino acid sequence of human RGMa precursor protein
SEQ ID NO: 2: Amino acid sequence of mouse RGMa precursor protein
SEQ ID NO: 3: Amino acid sequence of rat RGMa precursor protein
SEQ ID NO: 4: DNA sequence of human RGMa gene
SEQ ID NO: 5: Amino acid sequence of LCDR1 of anti-RGMa neutralizing antibody r116A3
SEQ ID NO: 6: Amino acid sequence of LCDR2 of anti-RGMa neutralizing antibody r116A3
SEQ ID NO: 7: Amino acid sequence of LCDR3 of anti-RGMa neutralizing antibody r116A3
SEQ ID NO: 8: Amino acid sequence of HCDR1 of anti-RGMa neutralizing antibody r116A3
SEQ ID NO: 9: Amino acid sequence of HCDR2 of anti-RGMa neutralizing antibody r116A3

SEQ ID NO: 10: Amino acid sequence of HCDR3 of anti-RGMa neutralizing antibody r116A3
SEQ ID NO: 11: Amino acid sequence of LCDR1 of anti-RGMa neutralizing antibody r70E
SEQ ID NO: 12: Amino acid sequence of LCDR2 of anti-RGMa neutralizing antibody r70E
SEQ ID NO: 13: Amino acid sequence of LCDR3 of anti-RGMa neutralizing antibody r70E
SEQ ID NO: 14: Amino acid sequence of HCDR1 of anti-RGMa neutralizing antibody r70E
SEQ ID NO: 15: Amino acid sequence of HCDR2 of anti-RGMa neutralizing antibody r70E
SEQ ID NO: 16: Amino acid sequence of the epitope of human RGMa
SEQ ID NO: 17: Amino acid sequence of LCDR1 of anti-RGMa neutralizing antibody 5F9
SEQ ID NO: 18: Amino acid sequence of LCDR2 of anti-RGMa neutralizing antibody 5F9
SEQ ID NO: 19: Amino acid sequence of LCDR3 of anti-RGMa neutralizing antibody 5F9
SEQ ID NO: 20: Amino acid sequence of HCDR1 of anti-RGMa neutralizing antibody 5F9
SEQ ID NO: 21: Amino acid sequence of HCDR2 of anti-RGMa neutralizing antibody 5F9
SEQ ID NO: 22: Amino acid sequence of HCDR3 of anti-RGMa neutralizing antibody 5F9
SEQ ID NO: 23: Amino acid sequence of LCDR1 of anti-RGMa neutralizing antibody 8D1
SEQ ID NO: 24: Amino acid sequence of LCDR2 of anti-RGMa neutralizing antibody 8D1
SEQ ID NO: 25: Amino acid sequence of LCDR3 of anti-RGMa neutralizing antibody 8D1
SEQ ID NO: 26: Amino acid sequence of HCDR1 of anti-RGMa neutralizing antibody 8D1
SEQ ID NO: 27: Amino acid sequence of HCDR2 of anti-RGMa neutralizing antibody 8D1
SEQ ID NO: 28: Amino acid sequence of HCDR3 of anti-RGMa neutralizing antibody 8D1
SEQ ID NO: 29: Amino acid sequence of LCDR1 of anti-RGMa neutralizing antibody AE12-1
SEQ ID NO: 30: Amino acid sequence of LCDR2 of anti-RGMa neutralizing antibody AE12-1
SEQ ID NO: 31: Amino acid sequence of LCDR3 of anti-RGMa neutralizing antibody AE12-1
SEQ ID NO: 32: Amino acid sequence of HCDR1 of anti-RGMa neutralizing antibody AE12-1
SEQ ID NO: 33: Amino acid sequence of HCDR2 of anti-RGMa neutralizing antibody AE12-1
SEQ ID NO: 34: Amino acid sequence of HCDR3 of anti-RGMa neutralizing antibody AE12-1
SEQ ID NO: 35: Amino acid sequence of LCDR3 of anti-RGMa neutralizing antibody AE12-1Y
SEQ ID NO: 36: Amino acid sequence of the epitope of human RGMa
SEQ ID NO: 37: Amino acid sequence of the epitope of human RGMa
SEQ ID NO: 38: Amino acid sequence of the epitope of human RGMa
SEQ ID NO: 39: Amino acid sequence of the epitope of human RGMa
SEQ ID NO: 40: Amino acid sequence of LCDR3 of anti-RGMa neutralizing antibody AE12-1F
SEQ ID NO: 41: Amino acid sequence of LCDR3 of anti-RGMa neutralizing antibody AE12-1H
SEQ ID NO: 42: Amino acid sequence of LCDR3 of anti-RGMa neutralizing antibody AE12-1L
SEQ ID NO: 43: Amino acid sequence of LCDR3 of anti-RGMa neutralizing antibody AE12-1V
SEQ ID NO: 44: Amino acid sequence of LCDR3 of anti-RGMa neutralizing antibody AE12-1I
SEQ ID NO: 45: Amino acid sequence of LCDR3 of anti-RGMa neutralizing antibody AE12-1K

Industrial Availability

The present invention is useful in prevention or treatment of peripheral neuropathies or pain symptoms associated with diseases having peripheral neuropathies or astrocytic damages, and thus has a high utility value in the pharmaceutical industry.

## Claims

1. An agent for preventing or treating a peripheral neuropathy, which agent comprises an RGM inhibiting substance.

2. The agent according to claim 1, wherein the RGM inhibiting substance is an RGMa inhibiting substance.

3. The agent according to claim 2, wherein the RGMa inhibiting substance is an anti-RGMa neutralizing antibody or a fragment thereof.

4. The agent according to claim 3, wherein the anti-RGMa neutralizing antibody is a humanized antibody.

5. The agent according to claim 3 or 4, wherein the anti-RGMa neutralizing antibody is an antibody recognizing an amino acid sequence selected from the group consisting of SEQ ID NOS: 16, 36, 37, 38 and 39.

6. The agent according to any one of claims 3 to 5, wherein the anti-RGMa neutralizing antibody is an antibody selected from the group consisting of:

(a1) an anti-RGMa neutralizing antibody which comprises
a light chain variable region comprising an LCDR1 comprising the amino acid sequence represented by SEQ ID NO: 5, an LCDR2 comprising the amino acid sequence represented by SEQ ID NO: 6 and an LCDR3 comprising the amino acid sequence represented by SEQ ID NO: 7, and
a heavy chain variable region comprising an HCDR1 comprising the amino acid sequence represented by SEQ ID NO: 8, an HCDR2 comprising the amino acid sequence represented by SEQ ID NO: 9 and an HCDR3 comprising the amino acid sequence represented by SEQ ID NO: 10;
(b 1) an anti-RGMa neutralizing antibody which comprises
a light chain variable region comprising an LCDR1 comprising the amino acid sequence represented by SEQ ID NO: 11, an LCDR2 comprising the amino acid sequence represented by SEQ ID NO: 12 and an LCDR3 comprising the amino acid sequence represented by SEQ ID NO: 13, and
a heavy chain variable region comprising an HCDR1 comprising the amino acid sequence represented by SEQ ID NO: 14, an HCDR2 comprising the amino acid sequence represented by SEQ ID NO: 15 and an HCDR3 comprising an amino acid sequence comprising SFG;
(c1) an anti-RGMa neutralizing antibody which comprises
a light chain variable region comprising an LCDR1 comprising the amino acid sequence represented by SEQ ID NO: 17, an LCDR2 comprising the amino acid sequence represented by SEQ ID NO: 18 and an LCDR3 comprising the amino acid sequence represented by SEQ ID NO: 19, and
a heavy chain variable region comprising an HCDR1 comprising the amino acid sequence represented by SEQ ID NO: 20, an HCDR2 comprising the amino acid sequence represented by SEQ ID NO: 21 and an HCDR3 comprising the amino acid sequence represented by SEQ ID NO: 22;
(d1) an anti-RGMa neutralizing antibody which comprises
a light chain variable region comprising an LCDR1 comprising the amino acid sequence represented by SEQ ID NO: 23, an LCDR2 comprising the amino acid sequence represented by SEQ ID NO: 24 and an LCDR3 comprising the amino acid sequence represented by SEQ ID NO: 25, and
a heavy chain variable region comprising an HCDR1 comprising the amino acid sequence represented by SEQ ID NO: 26, an HCDR2 comprising the amino acid sequence represented by SEQ ID NO: 27 and an HCDR3 comprising the amino acid sequence represented by SEQ ID NO: 28;

(e1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 31, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(f1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 35, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(g1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 40, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(h1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 41, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(i1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 42, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(j1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 43, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34;

(k1) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 44, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34; and

(11) an anti-RGMa neutralizing antibody which comprises

a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and

a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34.

**7.** The agent according to any one of claims 1 to 6, wherein the peripheral neuropathy is selected from the group consisting of a diabetic neuropathy; entrapment neuropathy selected from carpal tunnel syndrome, cubital ulnar neuropathy, peroneal nerve palsy and tarsal tunnel syndrome; familial amyloid polyneuropathy; toxic neuropathy; carcinomatous neuropathy; immune-mediated neuropathy selected from Guillain-Barre syndrome (GBS) and chronic inflammatory demyelinating polyneuropathy (CIDP); neuropathy associated with connective tissue diseases; Crow-Fukase syndrome (POEMS syndrome); hereditary neuropathy selected from Charcot-Marie-Tooth disease; postherpetic neuralgia; peripheral neuropathy associated with AIDS or Lyme disease; uremia; multifocal motor neuropathy; and vasculitis neuropathy.

**8.** The agent according to any one of claims 1 to 6, wherein the peripheral neuropathy is a diabetic neuropathy.

**9.** The agent according to claim 8, wherein the diabetic neuropathy is painful diabetic neuropathy and/or asymptomatic diabetic neuropathy.

**10.** The agent according to any one of claims 1 to 6, for use in prevention or treatment of pain symptoms associated with a disease having a peripheral neuropathy or an astrocytic damage.

**11.** The agent according to claim 10, wherein the disease having a peripheral neuropathy or an astrocytic damage is selected from the group consisting of:

a diabetic neuropathy; entrapment neuropathy selected from carpal tunnel syndrome, cubital ulnar neuropathy, peroneal nerve palsy and tarsal tunnel syndrome; familial amyloid polyneuropathy; toxic neuropathy; carcinomatous neuropathy; immune-mediated neuropathy selected from Guillain-Barre syndrome (GBS) and chronic inflammatory demyelinating polyneuropathy (CIDP);
neuropathy associated with connective tissue diseases; Crow-Fukase syndrome (POEMS syndrome); hereditary neuropathy selected from Charcot-Marie-Tooth disease; postherpetic neuralgia; peripheral neuropathy associated with AIDS or Lyme disease; uremia; multifocal motor neuropathy; vasculitis neuropathy;
neuromyelitis optica; and Alexander's disease.

**12.** The agent according to claim 11, wherein the disease having a peripheral neuropathy or an astrocytic damage is a diabetic neuropathy or neuromyelitis optica.

**13.** The agent according to claim 11, wherein the disease having a peripheral neuropathy or an astrocytic damage is diabetic neuropathy.

**14.** The agent according to claim 11, wherein the disease having a peripheral neuropathy or an astrocytic damage is neuromyelitis optica.

**15.** A method of preventing or treating a peripheral neuropathy, which comprises administering an effective amount of an RGMa inhibiting substance to a mammal in need thereof.

**16.** The method according to claim 15, wherein the peripheral neuropathy is diabetic neuropathy.

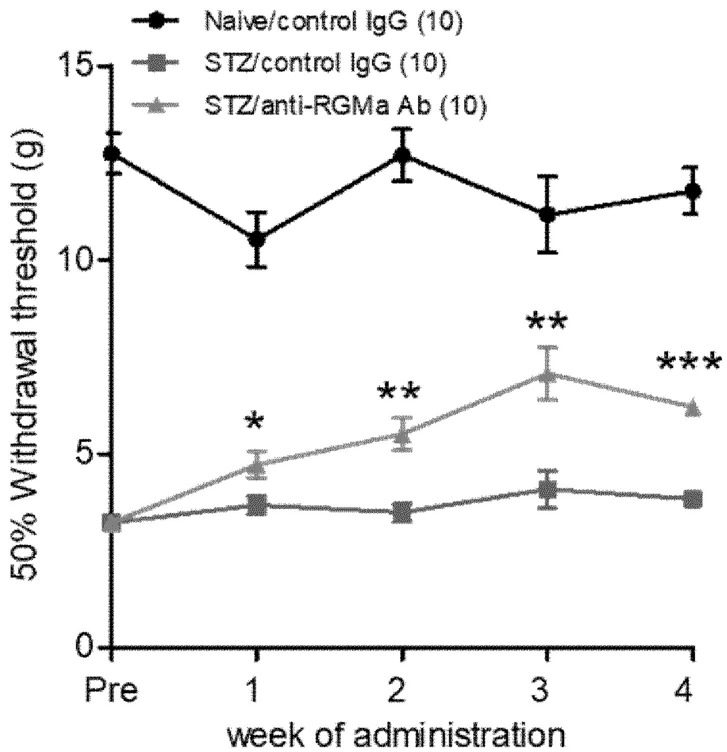


Each data indicates the mean ± S.E.M. (n=10)
Mann-Whitney U test *p<0.05, **p<0.001 vs STZ/control IgG.

Fig. 1

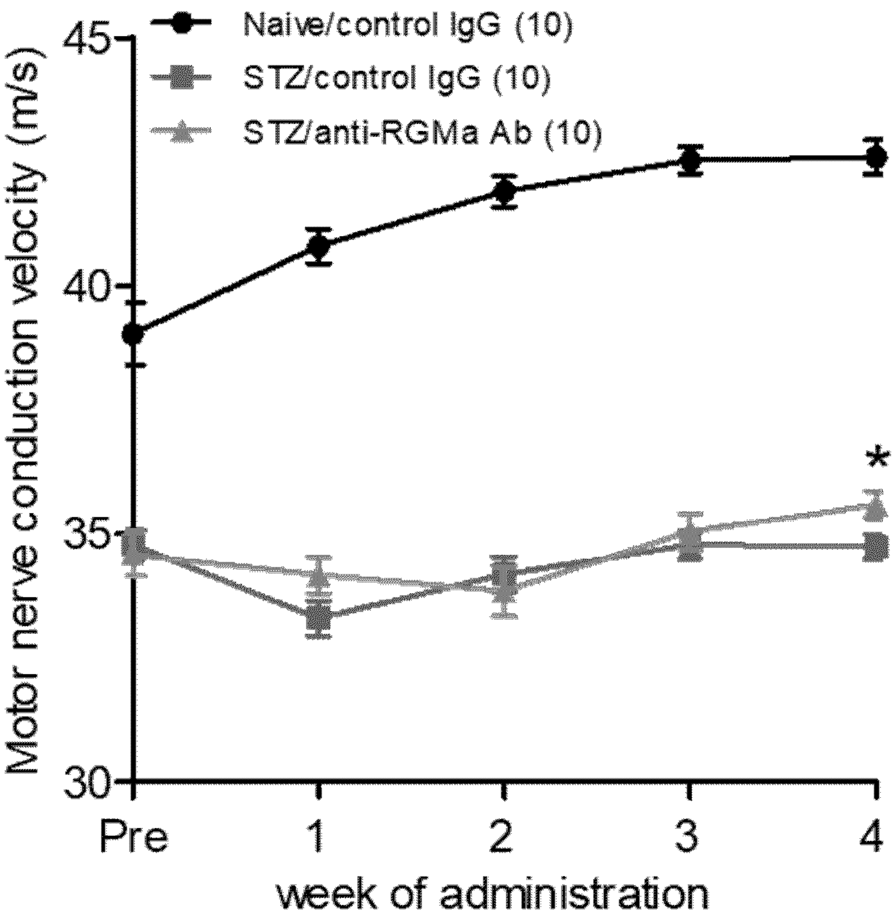


Each data indicates the mean ± S.E.M. (n=10)
Student's t test *p<0.05 vs STZ/control IgG.

Fig. 2

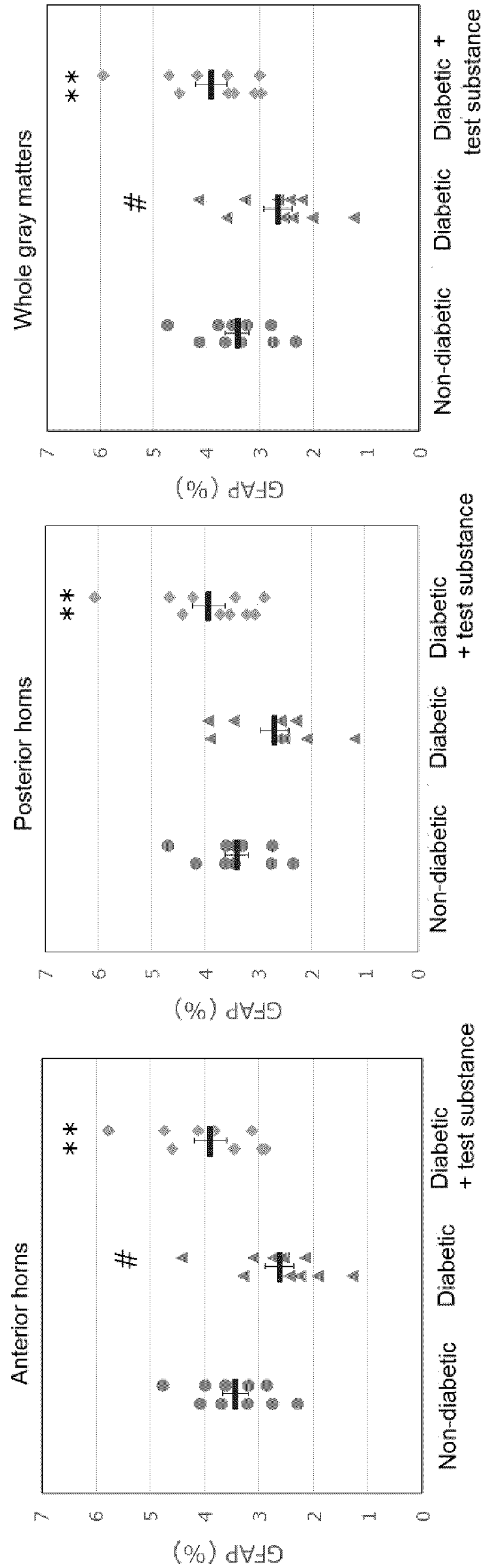
Anterior horns
Posterior horns
Whole gray matters
GFAP (%)
0
1
2
3
4
5
6
7
Non-diabetic
Diabetic
Diabetic + test substance
#
**

Fig. 3

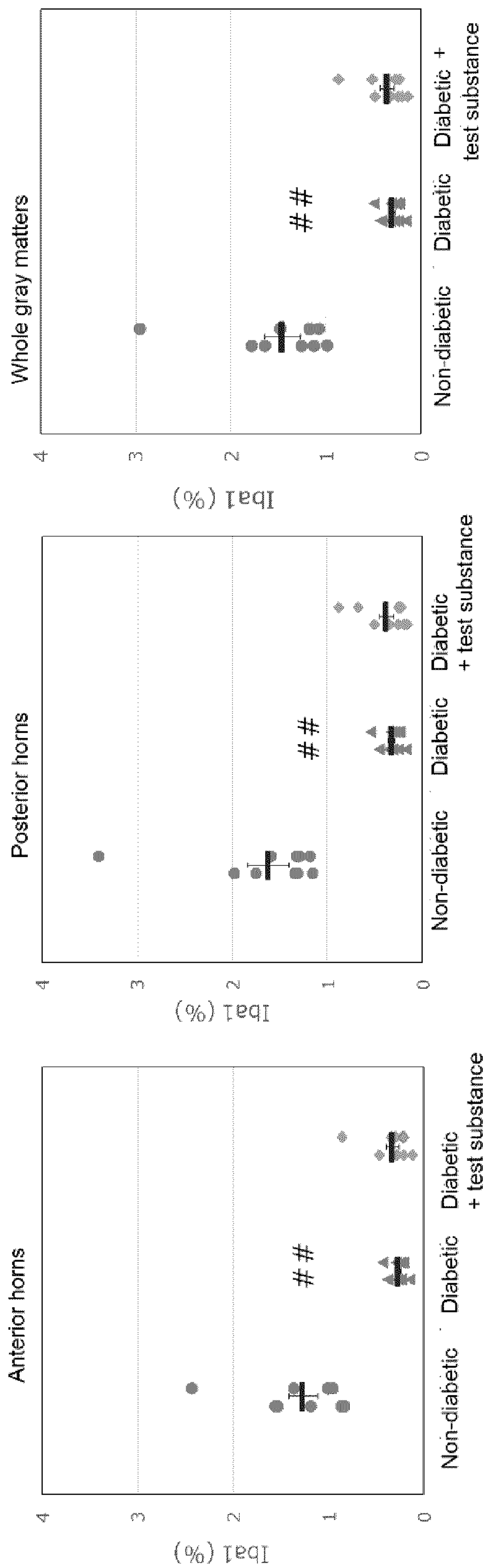
Anterior horns
Iba1 (%)
0
1
2
3
4
Non-diabetic
Diabetic
Diabetic
+ test substance
##
Posterior horns
Iba1 (%)
0
1
2
3
4
Non-diabetic
Diabetic
Diabetic
+ test substance
##
Whole gray matters
Iba1 (%)
0
1
2
3
4
Non-diabetic
Diabetic
Diabetic +
test substance
##

Fig. 4

## INTERNATIONAL SEARCH REPORT

International application No.
PCT/JP2019/027370

A. CLASSIFICATION OF SUBJECT MATTER
Int.Cl. A61K45/00(2006.01)i, A61K39/395(2006.01)i, A61P25/02(2006.01)i, A61P27/02(2006.01)i

According to International Patent Classification (IPC) or to both national classification and IPC

B. FIELDS SEARCHED

Minimum documentation searched (classification system followed by classification symbols)
Int.Cl. A61K45/00, A61K39/395, A61P25/02, A61P27/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | US 2013/0236477 A1 (EDGE, A.) 12 September 2013, claims, examples 3-4 & WO 2012/047706 A2 | 1-4, 7, 10, 11, 15<br>1-17 |
| X<br>Y | JP 2013-512942 A (ABBOTT GMBH & CO. KG) 18 April 2013, claims, examples & US 2011/0135664 A1, claims, examples & WO 2011/070045 A1 & EP 2510001 A1 | 1-17<br>1-17 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 October 2019 (01.10.2019) | 15 October 2019 (15.10.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/027370

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2015-508061 A (ABBVIE DEUTSCHLAND GMBH & CO. KG) 16 March 2015, claims, examples & US 2013/0330347 A1, claims, examples & WO 2013/112922 A1 & EP 2807192 A1 | 1-17<br>1-17 |
| X<br><br>Y | HARADA, K. et al., "Inhibition of RGMa alleviates symptoms in a rat model of neuromyelitis optica", Scientific Reports, January 2018, vol. 8, no. 34, pp. 1-9, abstract, fig. 1, 5 | 1-4, 10-12, 14-16<br>1-17 |
| X<br>Y | WO 2017/210278 A1 (ABBVIE INC.) 07 December 2017, claims, table 1, examples & JP 2019-517480 A & US 2017/0349653 A1 & EP 3464372 A1 | 10<br>1-17 |
| Y | WO 2016/175236 A1 (MITSUBISHI TANABE PHARMA CORPORATION) 13 November 2016, claims, examples & US 2018/0100012 A1, claims, examples & EP 3290441 A1 | 1-17 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005087268 A
- WO 2009106356 A
- WO 2013112922 A
- WO 2016175236 A
- US 4737456 A
- JP 4506458 A
- JP 2912618 B
- JP 62296890 A
- JP 4504365 A
- JP 7509137 A
- WO 9425585 A
- JP 6500233 A


### Non-patent literature cited in the description


- **DALLERAC G et al.** *Prog Neurobiol,* 2016, vol. 144, 48-67
- **GERKAU NJ et al.** *J Neurosci Res.,* 02 February 2017
- **ALAM Q et al.** *Curr Pharm Des.,* 2016, vol. 22, 541-8
- **YAMAZAKI et al.** *Neurosci Lett,* 1994, vol. 170, 195-197
- **STAHL, B. ; MULLER, B. ; VON BOXBERG, Y. ; COX, E.C. ; BONHOEFFER, F.** Biochemical characterization of a putative axonal guidance molecule of the chick visual system. *Neuron,* 1990, vol. 5, 735-743
- **MUELLER et al.** *Philos. Trans. R. Soc. Lond. B Biol. Sci.,* 2006, vol. 361, 1513-29
- **LIU, X. ; HASHIMOTO, M. ; HORII, H. ; YAMAGUCHI, A. ; NAITO, K. ; YAMASHITA, T.** Repulsive guidance molecule b inhibits neurite growth and is increased after spinal cord injury. *Biochem. Biophys. Res. Commun.,* 2009, vol. 382, 795-800
- **YAMASHITA, T. ; MUELLER, B.K. ; HATA, K.** Neogenin and repulsive guidance molecule signaling in the central nervous system. *Curr. Opin. Neurobiol,* 2007, vol. 17, 29-34
- **HATA, K. et al.** RGMa inhibition promotes axonal growth and recovery after spinal cord injury. *J. Cell Biol.,* 2006, vol. 173, 47-58
- **HARADA K. et al.** Inhibition of RGMa alleviates symptoms in a rat model of neuromyelitis optica. *Scientific Reports,* 2018, vol. 8 (34), 1-9
- **KABAT et al.** Sequences of Proteins of Immunological Interest. US Department of Health and Human Services, NIH, 1987
- Current Protocols in Molecular Biology. John Wiley & Sons, 1987
- Antibodies: A Laboratory Manual. Cold Spring Harbor Laboratory, 1988
- **KOHLER ; MILSTEIN et al.** *Nature,* 1975, vol. 256, 495
- *Nucleic Acids Research,* 1986, vol. 14, 1779
- *The Journal of Biological Chemistry,* 1982, vol. 257, 1516
- *Cell,* 1980, vol. 22, 197
- **HASHIGUCHI SHUHEI et al.** *The Journal of Japanese Biochemical Society,* 2010, vol. 82 (8), 710
- **D. J. KING.** Applications and Engineering of Monoclonal antibodies. T.J. International Ltd, 1998
- Monoclonal Antibody-Based Therapy of Cancer. Marcel Dekker Inc, 1998
- **CHARI et al.** *Cancer Res.,* 1992, vol. 152, 127
- **LIU et al.** *Proc Natl Acad Sci USA.,* 1996, vol. 93, 8681
- *Nature Genetics,* 1994, vol. 7, 13-21
- *Nature Genetics,* 1997, vol. 15, 146-156
- *Nature,* 1994, vol. 368, 856-859
- *Neuron,* 09 September 2010, vol. 67, 834-846
- **CHAPLAN, S.R. ; BACH, F.W. ; POGREL, J.W. ; CHUNG, J.M. ; YAKSH, T.L.** Quantitative assessment of tactile allodynia in the rat paw. *J. Neurosci. Methods,* 1994, vol. 53, 55-63